# EUROPEAN PATENT SPECIFICATION

(11) **EP 2 496 715 B1**
(45) Date of publication and mention of the grant of the patent: **27.01.2016**
(21) Application number: 10781757.9
(22) Date of filing: 03.11.2010
(51) Int. Cl.: C12Q 1/68

(54) **QUANTITATIVE NUCLEASE PROTECTION SEQUENCING (QNPS)**
QUANTITATIVE NUKLEASESCHUTZ-SEQUENZIERUNG (QNPS)
SÉQUENÇAGE QUANTITATIF UTILISANT UNE PROTECTION CONTRE LA NUCLÉASE (QNPS )

(30) Priority: 03.11.2009 US 257678 P
(43) Date of publication of application: 12.09.2012
(73) Proprietor: HTG Molecular Diagnostics, Inc., Tuscon, AZ 85706 (US)
(72) Inventor: SELIGMANN, Bruce, Tucson, AZ 85706 (US)
(74) Representative: Gowshall, Jonathan Vallance
(86) International application number: PCT/US2010/055289
(87) International publication number: WO 2011/056863

(56) References cited:
- US-A1- 2004 086 892
- US-A1- 2007 224 613
- US-A1- 2008 268 451
- MARTEL RALPH R ET AL: "Multiplexed screening assay for mRNA combining nuclease protection with luminescent array detection", ASSAY AND DRUG DEVELOPMENT TECHNOLOGIES, MARY ANN LIEBERT, INC. PUBLISHERS, US, vol. 1, no. 1, 1 November 2002 (2002-11-01), pages 61-71, XP002484291, ISSN: 1540-658X, DOI: DOI:10.1089/154065802761001310
- ROBERTS ROBIN A ET AL: "Quantitative nuclease protection assay in paraffin-embedded tissue replicates prognostic microarray gene expression in diffuse large-B-cell lymphoma", LABORATORY INVESTIGATION, NATURE PUBLISHING GROUP, US, vol. 87, no. 10, 1 October 2007 (2007-10-01), pages 979-997, XP002484290, ISSN: 0023-6837, DOI: DOI:10.1038/LABINVEST.3700665
- COPPEE ET AL: "Do DNA microarrays have their future behind them?", MICROBES AND INFECTION, ELSEVIER, PARIS, FR, vol. 10, no. 9, 1 July 2008 (2008-07-01), pages 1067-1071, XP025838955, ISSN: 1286-4579, DOI: DOI:10.1016/J.MICINF.2008.07.003 [retrieved on 2008-07-09]
- TENG XIAOKUN ET AL: "Perspectives of DNA microarray and next-generation DNA sequencing technologies.", SCIENCE IN CHINA. SERIES C, LIFE SCIENCES / CHINESE ACADEMY OF SCIENCES JAN 2009 LNKD- PUBMED:19152079, vol. 52, no. 1, January 2009 (2009-01), pages 7-16, XP007916654, ISSN: 1006-9305
- SHENDURE J ET AL: "Next-generation DNA sequencing", NATURE BIOTECHNOLOGY, NATURE PUBLISHING GROUP, NEW YORK, NY, US, vol. 26, no. 10, 1 October 2008 (2008-10-01), pages 1135-1145, XP002572506, ISSN: 1087-0156, DOI: DOI:10.1038/NBT1486 [retrieved on 2008-10-09]
- Illumina: "Multiplexed Sequencing with the Illumina Genome Analyzer System", , 8 January 2009 (2009-01-08), pages 1-4, Retrieved from the Internet: URL:http://res.illumina.com/documents/prod ucts/datasheets/datasheet_sequencing_multi plex.pdf [retrieved on 2013-07-01]
- Daniel N Frank: "BARCRAWL and BARTAB: software tools for the design and implementation of barcoded primers for highly multiplexed DNA sequencing", BMC Bioinformatics, vol. 10, no. 1, 1 January 2009 (2009-01-01), page 362, XP55003933, ISSN: 1471-2105, DOI: 10.1186/1471-2105-10-362
- Kun Zhang ET AL: "Digital RNA allelotyping reveals tissue-specific and allele-specific gene expression in human", Nature Methods, vol. 6, no. 8, 1 August 2009 (2009-08-01), pages 613-618, XP055068957, ISSN: 1548-7091, DOI: 10.1038/nmeth.1357
- J. B. Li ET AL: "Multiplex padlock targeted sequencing reveals human hypermutable CpG variations", Genome Research, vol. 19, no. 9, 1 September 2009 (2009-09-01), pages 1606-1615, XP055042008, ISSN: 1088-9051, DOI: 10.1101/gr.092213.109

## Description

The present invention generally relates to methods for performing quantitative nuclease protection sequencing (qNPS) in the identification and detection of nucleic acid targets. More specifically, the present invention provides methods for analyzing nucleic acids from biological samples using sequencing.

The present invention provides a new approach, quantitative Nuclease Protection Sequencing (qNPS™), for addressing several challenges that face sequencing and which provides improvements for research and diagnostic applications. The method uses a lysis-only nuclease protection assay to generate DNA (or other synthetic) probes for sequencing, which can be sequenced themselves or coupled to (a) gene-specific tags to permit the identification of the gene without necessitating the sequencing of the nuclease protection probe itself and/or (b) experiment-specific tags, permitting samples from different patients to be combined into a single run. The disclosed qNPS makes sequencing of fixed or insoluble samples as well as all types of other samples possible and affordable as a research and discovery tool and as a diagnostic test.

Methods for sequencing on current systems (e.g. 454, Solexa, SOLID) and on next generation platforms (e.g. single molecule sequencing) are further disclosed. qNPS provides a focused or targeted sequencing capability for research and diagnostics that, among other things,: i) provides a low cost/sample; ii) provides high sample throughput; iii) reduces sequencing run time and simplifies data analysis; iv) permits the efficient sequencing of target genes without interference from the background of other (e.g. pathogen from host) genes; v) provides a precise way to measure signature sets of gene expression, expressed single nucleotide polymorphisms (SNPs), DNA SNPs, DNA methylation, rRNA, miRNA, mutations, etc., that are useful as biomarkers; vi) enables sequencing from all sample types, in particular from fixed tissues, such as formalin fixed tissues or fixed, intracellular stained and sorted samples; and vii) greatly simplifies the complexity of the sample that is sequenced from whole genes to just nuclease protection probes or the target sequence protected by that probe.

Animal tissues and clinical samples are typically preserved by fixation in the form of paraffin-embedded formalin-fixed (FFPE) tissue. Thus, a commercially viable diagnostic assay of tissue gene expression and DNA must be able to use FFPE. Furthermore, millions of such samples are archived at clinical centers and hospitals, and the corresponding treatment modalities and clinical outcomes are known. FFPE samples therefore represent an invaluable resource for rapidly and efficiently identifying diagnostic biomarkers and then developing and validating prognostic and diagnostic assays.

US 2008/268451 A1 discloses a quantitative nuclease protection assay which uses an ArrayPlate.

Several challenges face sequencing for research and diagnostic applications. The disclosed quantitative Nuclease Protection Sequencing (qNPS) method uses a lysis-only nuclease protection assay to generate (e.g., DNA) probes for sequencing, which can be sequenced directly or which can be coupled to, for example, (i) gene-specific tags to permit the identification of the gene sequence being measured without need to sequence the nuclease protection probe itself; and/or ii) to experiment-specific tags, one unique tag for each separate sample so that different samples (e.g., from different patients or from different treatments or experiments) can be combined into a single sequencing run but remain differentiable after having been sequenced. qNPS provides a sequencing capability that, among other things,: i) provides a low cost/sample; ii) provides high sample throughput; iii) reduces sequencing run time and simplifies data analysis; iv) permits the efficient sequencing of target genes without interference from the background of non-target genes or gene sequences, including for instance the sequencing of pathogen genes from host tissue, or of graft tissue without interference of the host tissue genome; v) provides a precise way to measure signature sets of gene expression, expressed single nucleotide polymorphisms (SNP's), DNA SNP's, DNA methylation, all RNA including miRNA, rRNA, mutations or other nucleotide targets that are useful as biomarkers; vi) enables sequencing from all samples including in particular fixed tissues, such as formalin fixed tissues or hematoxylin and eosin (H&E) stained tissues, or glutaraldehyde fixed tissues such as fixed, intracellular stained and sorted cells; and vii) greatly simplifies the complexity of the sample that is sequenced from whole genes to just nuclease protection probes.

The invention is defined in the claims.

In one aspect, the present disclosure provides methods for the current generation of, e.g., 454, Solid and Solexa sequencers, and for the next generation of single molecule sequencers and beyond. While many of these systems have multiple channels permitting multiple samples to be sequenced in parallel, the cost per sequencing run is $7,000 to $9,000, and the run can last several days. Single molecule sequencers such as PacBio may offer costs on the range of $100 to $200/sample, but this is still expensive when sample preparation costs are added. A way to lower cost per sample and increase sample throughput is to test multiple samples in each sequencing run, within each channel of multichannel sequencers, using a sequencible "tag" to identify the molecules sequenced from each experiment - referred to as an "experiment tag". Shortening the sequence read length can increase efficiency. Sequencing just the nuclease protection probe rather than the entire gene or gene fragments, or using a short, unique gene tag to identify the target sequence achieves this efficiency for applications where sequencing is used to identify and quantify gene levels or presence (but not to identify unknown differences in gene sequence). Use of gene tags also simplifies nuclease protection probe design because the end accessible to sequencing does not have to be unique. However, the nuclease protection probes or target oligonucleotide protected by the probes can be directly sequenced without use of gene tags. In this case the presence of variations in the target sequence can also be identified where they result in S1 cleavage of or partial hydrolysis of the nuclease protection probes, resulting in a pattern of resultant partial probe sequences or when the protected portion of the target oligonucleotide is sequenced. The process can also be designed to include identification of the mutation(s). This is discussed further herein.

Sequencing is very powerful for identifying differences in genomic DNA that may pre-dispose persons to certain diseases or warn of adverse drug metabolism. However, a great deal of development remains to implement sequencing methods useful for diagnostics to identify the patients' condition and prognosticate response to therapy which will require, for instance, the assessment of gene expression, miRNA levels, and DNA methylation states and other mutations from clinically relevant sample types. Gene sequencing companies have not focused on this area in their commercial quest to provide sequencing of the genome at lower and lower cost.

Sequencing from fixed, such as paraffin-embedded formalin-fixed (FFPE), tissue has been problematic and difficult, yet clinical samples are typically preserved by fixation, in the form of FFPE tissue. Thus, whether the interest is to identify putative biomarkers or disease and drug mechanisms, or to develop and then apply as the basis for a commercially viable diagnostic assay of tissue gene expression and DNA, the assay must be able to use FFPE. Furthermore, millions of such samples are archived at clinical centers and hospitals, and the corresponding treatment modalities and clinical patient outcomes of the FFPE donors are known. FFPE and other fixed samples therefore represent an invaluable resource for rapidly and efficiently identifying drug targets, disease markers and pathways and diagnostic biomarkers and then developing and validating prognostic and diagnostic assays, or for identifying genes and changes in expression of methylation states or mutations associated with disease progression or drug activity. Sequencing DNA and RNA from FFPE is not just problematic for sequencing, but also for array-based methods and PCR, and probably for the same reason--a significant portion of the genomic DNA, and transcriptomic RNA, is cross-linked to the tissue. This cross-linking must be reversed and the target genes recovered for processing and analysis. Total RNA recovered from FFPE is typically partially degraded, whether due to fixation or the process of extracting the RNA from the FFPE. In the research setting, samples that are too degraded for analysis can simply be discarded, but in the diagnostic setting, discarding a patient's sample is not acceptable. Thus, while the power of sequencing is recognized, the application to FFPE in a research setting or in particular, a diagnostic setting, is quite challenging. From the research perspective the information content of formalin fixed paraffin embedded (FFPE) tissue remains locked in the vast archives of these samples waiting for a precise and simple method of analysis. All the above apply to all nucleic acids, DNA, RNA, tRNA, rRNA, miRNA, etc. and mutations within those sequences.

Another challenge confronting sequencing applications is the cost per sample. Currently, a sequencing run can cost $7,000 to $10,000. Whether the need is to sequence different patient samples or to sequence samples from different experiments, testing each separately, even if a different sample is tested in each channel of an (e.g., 454 or Solexa) instrument, the cost per sample is ∼$1,000. The disclosed invention provides the ability to combine different experimental or patient samples into a single run, within the same instrument channel, using experimental tags attached to each molecule. These are sequenced to uniquely identify all the molecules from each single experiment or patient sample that were combined into a single sequencing sample from one another. For instance, by combining the samples of 100 patients (the qNPS products from each patient sample, each marked with a different unique experimental tag) into a single e.g., 3-day run, the sequencing cost per sample is only ∼$10. With costs at this level for measuring 100's of genes/sample, diagnostic tests and routine experiments or screening assays become affordable even after adding on the cost of processing the sample (e.g., collecting it, processing it, etc.).

Not only does the use of experiment tags reduce the cost/sample, but they also enable high sample throughput, e.g., by permitting 100's or 1,000's of different experiments to be sequenced in a single run, within a single channel. For example, pooling 100 samples per channel, 8,000 samples could be tested in a single run of an 8-channel sequencer. This enables, for instance, high throughput screening applications, across many gene targets/sample.

Another advantage of the qNPS process is the simplified data analysis that results. Because only target molecules are hybridized to the nuclease protection probes, the remaining genomic DNA and RNA in the sample is either destroyed or made inaccessible to sequencing (e.g., by not having sequencing adaptor molecules ligated onto them), leaving only the quantitative set of nuclease protection probes or their protected target oligonucleotides to be sequenced. Because the sequence of these probes and targets is known, the reference sequence database need only consist of those sequences, not the entire genome. Furthermore, if a standard set of gene identifier tags is incorporated into the sequenced NPP adduct, and then the deconvolution of sequencing information is even further simplified. In essence, sequence analysis can be reduced to "counting" the number of each identified known sequence or partial sequence of the synthetic nuclease protection probes and derived sequencible adducts or the target oligonucleotides and identifying any differences in the sequences of the target oligonucleotides.

A further advantage of this is that rare molecules can be sequenced, or for instance target molecules from a pathogen can be sequenced from host tissue without the burdensome sequencing of the host genome. Just as important, when sequencing is used to quantitatively measure the level of expressed genes, it is important to be able to measure genes that are expressed at the level of thousands of copies/cell as well as genes that are measured at a level of only one copy per cell. By eliminating the background of the whole genome, and focusing just on the target genes of interest, and in fact reducing the target gene itself to a short sequence (e.g., the 50 bases of the nuclease protection probe), or to an even shorter gene identifier tag, the efficiency of sequencing is increased and the dynamic range to measure genes of vastly different abundance is increased.

Sequencing just the nuclease protection probe or use of gene identifier tags also reduces read time, permitting sequencing results to be obtained much faster.

Also, because the qNPS protocol utilizes lysis of the sample, and does not require extraction or (e.g., for gene expression) reverse transcription, it can be fully and simply automated. This is a necessity for high throughput screening and is also an asset for diagnostic assays or general laboratory assays. Furthermore, the lysed sample contains all target molecules, such as all the mRNA and all the miRNA. Extraction protocols frequently lose a portion of one or the other of these, or require the separation of RNA from DNA. To be clear, qNPS can be performed on any sample, including (e.g.) purified RNA, miRNA, DNA or cDNA.

All types of target molecules can be measured by qNPS. Examples are DNA, DNA single nucleotide polymorphisms (SNP's), methylated DNA levels, mRNA expression, mRNA SNP's, miRNA levels, rRNA levels, siRNA, tRNA, gene fusions or other mutations, protein-bound DNA or RNA, and also cDNA, etc. Anything to which a nuclease protection probe can be designed to hybridize can be quantified and identified by sequencing, even though the target molecules themselves are never sequenced and often most preferably are destroyed. The nuclease protection probe protects the target molecule from nuclease for sequencing, and the gene tags and experiment tags can be attached to the target molecule rather than to the nuclease protection probes. In either case, the target molecules are thereafter dispensable optionally, as are the NPPs.

### Sequencing

"Sequencing," as is used herein, means to determine the primary structure (or primary sequence) of an unbranched biopolymer. Sequencing results in a symbolic linear depiction known as a sequence which succinctly summarizes much of the atomic-level structure of the sequenced molecule, for example, a polynucleotide or a polypeptide. Wherein the molecule is a polynucleotide, such as, for example, RNA or DNA, sequencing can be used to obtain information about the molecule at the nucleotide level, which can then be used in deciphering various secondary information about the molecule itself and/or the polypeptide encoded thereby.

When the polynucleotide is an RNA molecule, owing to the instability of the molecule and its propensity towards nuclease (for example, RNase) degradation, it is conventionally preferable to first reverse transcribe the sample to generate DNA fragments, which can then be sequenced by any of the methods described herein. This remains an option for this invention. However, qNPS avoids the need for reverse transcription, instead converting the target RNA sequence into a complementary DNA probe sequence through hybridization and nuclease activity. As is understood in the art, it is sometimes desirable to sequence RNA molecules rather than the gene sequences which encode the RNA, since, RNA molecules are not necessarily co-linear with their DNA template. And some organisms are RNA, such as RNA viruses. For example, intron excision and splicing are two events that contribute towards the non-linearity between the two polynucleotide species. In other embodiments of the present invention, the whole transcriptome of a cell or a tissue may be analyzed using additional methods that are known in the art.

Any sequencing method can be employed in this invention.

DNA sequencing is the process of determining the nucleotide order of a given DNA fragment. Thus far, most DNA sequencing has been performed using the chain termination method (developed by Frederick Sanger). This technique uses sequence-specific termination of a DNA synthesis reaction using modified nucleotide substrates. In chain terminator sequencing, extension is initiated at a specific site on the template DNA by using a short oligonucleotide 'primer' complementary to the template at that region. The oligonucleotide primer is extended using a DNA polymerase, an enzyme that replicates DNA. Included with the primer and DNA polymerase are the four deoxynucleotide bases (DNA building blocks), along with a low concentration of a chain terminating nucleotide (most commonly a di-deoxynucleotide). Limited incorporation of the chain terminating nucleotide by the DNA polymerase results in a series of related DNA fragments that are terminated only at positions where that particular nucleotide is used. The fragments are then size-separated by electrophoresis in a slab polyacrylamide gel, or more commonly now, in a narrow glass tube (capillary) filled with a viscous polymer.

An alternative to the labeling of the primer is to label the terminators instead, commonly called 'dye terminator sequencing'. The major advantage of this approach is the complete sequencing set can be performed in a single reaction, rather than the four needed with the labeled-primer approach. This is accomplished by labeling each of the dideoxynucleotide chain-terminators with a separate fluorescent dye, which fluoresces at a different wavelength. This method is easier, cheaper, and quicker than the dye primer approach.

Pyrosequencing has been commercialized by Biotage (for low throughput sequencing) and 454 Life Sciences (for high-throughput sequencing) among others. The latter platform sequences roughly 100 megabases in a 7-hour run with a single machine. In the array-based method (commercialized by 454 Life Sciences), single-stranded DNA is annealed to beads and amplified via EmPCR. These DNA-bound beads are then placed into wells on a fiber-optic chip along with enzymes which produce light in the presence of ATR When free nucleotides are washed over this chip, light is produced as ATP is generated when nucleotides join with their complementary base pairs. Addition of one (or more) nucleotide(s) results in a reaction that generates a light signal that is recorded by the CCD camera in the instrument. The signal strength is proportional to the number of nucleotides, for example, homopolymer stretches, incorporated in a single nucleotide flow.

Current sequencers (Solexa, 454, Solid) capture target sequences onto a sequencing chip or bead and then amplify before sequencing. Next generation single molecule sequencing does not use amplification after capture. Adaptor sequences or Poly A tails are used for capture. Alternatively, there may be no capture step. Instead, (e.g.) captured polymerase can be used to capture and sequence the passing oligonucleotide.

Sequencing by 454 or Solexa typically involves library preparation, accomplished by random fragmentation of DNA, followed by *in vitro* ligation of common adaptor sequences. For qNPS, the step of random fragmentation of DNA can be by-passed and the in vitro ligation of adaptor sequences can be to the nuclease protection probe, or to the gene tag or experiment tag for the nuclease protection probe. Shendure and Ji (2008) review sequencing methods, and what follows briefly summarizes the 454 and Solexa systems. For 454 and Solexa, the generation of clonally clustered amplicons to serve as sequencing features, using emulsion PCR or bridge PCR, respectively. What is common to these methods is that PCR amplicons derived from any given single library molecule end up spatially clustered, either to a single location on a planar substrate (Solexa, *in situ* polonies, bridge PCR), or to the surface of micron-scale beads (454, emulsion PCR), which can be recovered and arrayed (emulsion PCR). The sequencing process itself consists of alternating cycles of enzyme-driven biochemistry and imaging-based data acquisition. These platforms rely on sequencing by synthesis, that is, serial extension of primed templates. Successive iterations of enzymatic interrogation and imaging are used to build up a contiguous sequencing read for each array feature. Data are acquired by imaging of the full array at each cycle (e.g., of fluorescently labeled nucleotides incorporated by a polymerase).

For 454, a sequencing primer is hybridized to the universal adaptor at the appropriate position and orientation, immediately adjacent to the start of unknown sequence or qNPS sequencible adduct such as the nuclease protection probe or gene or experiment tag. Sequencing is performed by pyrosequencing. Amplicon-bearing beads are pre-incubated with *Bacillus stearothermophilus* (*Bst*) polymerase and single-stranded binding protein, and then deposited on to a microfabricated array of picoliterscale wells, one bead per well, rendering this biochemistry compatible with array-based sequencing. Smaller beads are also added, bearing immobilized enzymes also required for pyrosequencing (ATP sulfurylase and luciferase). During the sequencing, one side of the semi-ordered array functions as a flow cell for introducing and removing sequencing reagents. The other side is bonded to a fiber-optic bundle for CCD-based signal detection. At each cycle, a single species of unlabeled nucleotide is introduced. For sequences where this introduction results in incorporation, pyrophosphate is released via ATP sulfurylase and luciferase, generating a burst of light detected by the CCD for specific array coordinates. Across multiple cycles, the pattern of detected incorporation events reveals the sequence of templates represented by individual beads.

For Solexa, amplified sequencing features are generated by bridge PCR. Both forward and reverse PCR primers are tethered to a solid substrate by a flexible linker, such that all amplicons arising from any single template molecule during the amplification remain immobilized and clustered to a single physical location on an array. The bridge PCR is somewhat unconventional in relying on alternating cycles of extension with *Bst* polymerase and denaturation with formamide. The resulting 'clusters' each consist of -1,000 clonal amplicons. Several million clusters can be amplified to distinguishable locations within each of eight independent 'lanes' that are on a single flow-cell (such that eight independent experiments can be sequenced in parallel during the same instrument run). After cluster generation, the amplicons are linearization and a sequencing primer is hybridized to a universal adaptor sequence flanking the region of interest. Each cycle of sequence interrogation consists of single-base extension with a modified DNA polymerase and a mixture of four nucleotides. These nucleotides are 'reversible terminators', in that a chemically cleavable moiety at the 3' hydroxyl position allows only a single-base incorporation to occur in each cycle, and one of four fluorescent labels, also chemically cleavable, corresponds to the identity of each nucleotide. After single-base extension and acquisition of images in four channels, chemical cleavage of both groups sets up for the next cycle. Read-lengths up to 36 bp are currently routinely performed. This dictates a target length for the qNPS adducts (seven sequencing start and experiment tag bases, generic capture sequence 2 of ten to fifteen bases, and five gene tag bases).

Other methods of sequencing are or will be developed, and one skilled in the art can see that the qNPS probes, gene tags, and experiment tags and analogous sequencible adducts (as discussed below) will be suitable for sequencing on these systems.

### qNPS

qNPS is a fundamentally different approach to sequencing that uses a quantitative Nuclease Protection Assay to stoichiometrically convert unstable RNA or other target molecules from tissue lysates (or purified RNA or DNA), even when cross linked, into stable single-stranded DNA targets (nuclease protection probes) that can be recovered in solution without capture or separation, by use of the nuclease protection step and (as necessary) treatment with base to dissociate the nuclease protection probes from protecting target molecules, and in the case of RNA, hydrolyze the RNA target. The amounts of the nuclease protection probes remaining after S1 nuclease hydrolysis are then determined by sequencing which can include sequencing of the probes themselves and detection of the mentioned partial probe sequences. Currently the products of this nuclease protection assay (commonly referred to as qNPA™, H.T.G., Inc., Tucson, AZ 85706) are measured using a highly sensitive array-based read-out, thus providing a measurement of the level of each target gene. See, e.g., US 6,232,066, US 6,238,869, WO 2008-121927. A number of publications have also described applications of qNPA (Altar et al, 2208 and 2009, Kris et al, Martel et al 2002 and 2004, Roberts et al, Rimsza et al, Sawada et al, and Seligmann et al). The qNPS assay can be configured in many different ways but all utilize the concept of producing a NPP that survives a nuclease reaction (e.g., S1 digestion) as the central adduct that is sequenced, or producing an adduct, part or all of which that can be sequenced to specifically identify and quantify the NPP or mentioned remnant nuclease protection probe sequences, and hence the target gene. The process will also identify the existence of any alterations in the portion of the target gene measured by the nuclease protection probe or between multiple nuclease protection probes targeting the same gene.

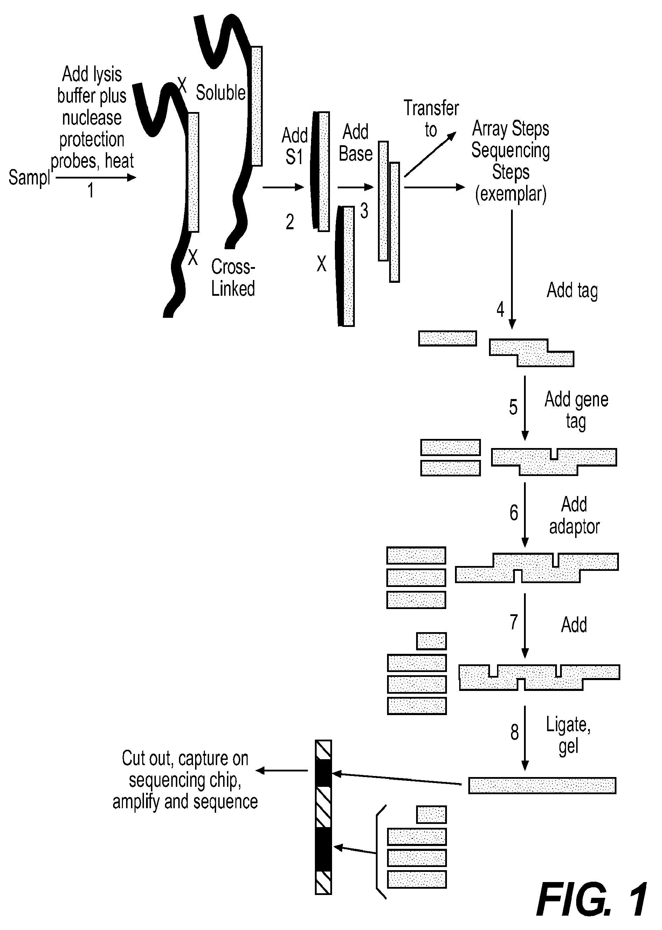

The production of the nuclease protection probe (NPP) from sample for the qNPS assay is carried out as depicted in Figure 1, similar to the method published for qNPA (Roberts et al, 2007; Martel et al 2002 and 2004). The assay comprises one or more different nuclease protection probe(s) designed to be specific for each different target. Thus, the measurement of 100 genes requires the design and synthesis of 100 different nuclease protection probes, one per gene or several hundred different NPP, several/gene. These are most preferably comprised of DNA, and can be about 10 to about 100 or about 200 or more bases in length, but more preferably 20 to 75 bases in length, and most preferably 20 to 50 bases in length. Figure 1 Step 1 depicts the addition of a lysis reagent to the sample plus nuclease protection probes (NPP) in a great excess. In this figure only a single species of target molecule (RNA) and nuclease protection probe is depicted but one RNA target molecule is indicated as cross-linked to the tissue (by the "X's") and another as soluble. The assay can also be run on extracted (or purified) RNA or other target molecules. The probes are designed to be specific for the target molecule, and to have similar Tm's but sufficiently unique sequences to permit the probes to be differentiated by sequencing, or to support specific hybridization for attachment of gene tags. The sample is preferably heated at around 95°C or about 105°C for approximately ten minutes to denature the target molecules, rendering them single stranded and available for hybridization. Using different denaturation solution, this denaturation temperature can be modified, so long as the combination of temperature and buffer composition leads to formation of single stranded target DNA or RNA). Then the sample is incubated at a specified temperature for a period of time (e.g., for 50-mer nuclease protection probes, 6 hr at 60°C) to permit hybridization of probes to the target molecules. A nuclease (e.g., S1 nuclease) or cocktail of nucleases is added and incubation carried out (e.g., for 60 min at 50°C for 50-mer nuclease protection probes) during which time the nuclease destroys all the excess nuclease protection probes that are not hybridized to target molecule (and thus are unprotected), all the non-target molecules in the sample (e.g., RNA or DNA), and the overhang single stranded region of the target molecules, and if desired cleaves the probe at bases which are not paired with the target sequence, leaving a stoichiometric amount of target molecule/nuclease protection probe duplex (Step 2) or partial probe duplex (where the mentioned unpairing exists). See below. In this figure the "X""s represent the cross-linking of target molecule to tissue that occurs from fixation. The nuclease protection probes hybridize to the cross-linked target molecule without the need to reverse cross-linking. Conditions can be selected such that single nucleotide differences leading to an unpaired base is not cleaved, or a nuclease can be used which just cleaves unpaired bases up to the ends of the hybridized nuclease protection probe, such as an exonuclease.

After nuclease treatment the probes may still be associated with cross-linked target molecule sequences. However, in Step 3 base is added, and the sample is heated to 95°C. This dissociates the target molecule/nuclease protection probe dimers, leaving the nuclease protection probe in a single stranded state, and in the case of RNA hydrolyzes the RNA target molecules.

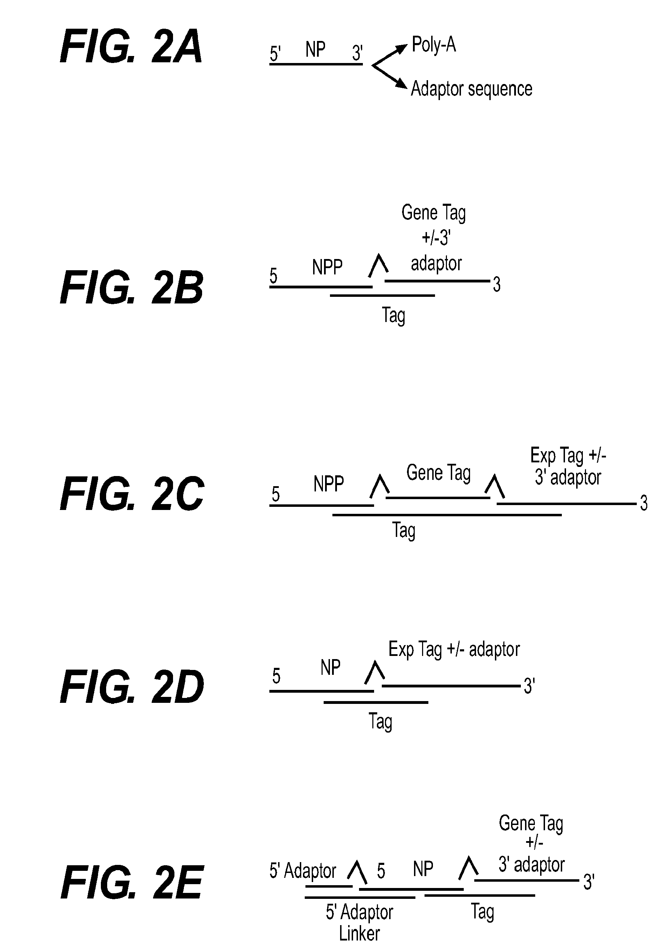

For qNPS the steps after this point can vary, depending on how the nuclease protection probe is going to be sequenced. The different adducts formed from the NPP are depicted in successive figures. If no gene tag or experiment tag is to be used, then the probes can be directly ligated with adaptor molecules suitable for the sequencing system (or a poly A tail can be added using, e.g., terminal deoxynucleotidyl transferase, Tdt), and used for sequencing (Figure 2A). Figure 1 (steps 4 through 8) and Figure 2 depict the addition of (and incubation with) an excess amount of tag linkers for each nuclease protection probe at a temperature that permits hybridization. Several possible sequencible adducts can be formed based on the use of the tag linker. For instance, 25 bases of the tag linker can be designed to be complementary with the 3' end of one specific nuclease protection probe, and thus will hybridize to that probe (step 4). The remainder of the tag linker can be designed to hybridize (and thus capture at the 3' end of the nuclease protection probe) a gene tag sequence (Figure 2B) and/or (optionally) the generic (or experiment specific sequence) portion of an experiment tag sequence (Figure 2C), or just the generic (or specific) portion of an experiment tag (Figure 2D), after the addition of excess amounts of these tags (Figure 1 Sep 5), followed by incubation at a temperature that permits their hybridization to the tag linker. Note that these steps can be combined or carried out separately. In the case where the sequencer requires an adaptor capture sequence at an end, or at each end, of the molecule to be sequenced, the tag linker can be extended the full length of the nuclease protection probe and further to include a sequence that is complementary to the (e.g., 5') adaptor sequence. However, more preferably, a second adaptor linker is added that hybridizes to the 5' end of the nuclease protection probe and contains a sequence that is complimentary to the 5' adaptor sequence (Step 6), and then that adaptor sequence is added (Step 7). In this same case the gene tag, or the experiment tag, whichever is the 3' pent-ultimate sequence, can be synthesized with the 3' adaptor sequence for sequencing. After the complete adduct is hybridized together the sequences can be ligated together using, for example, T4 DNA ligase (or a non enzymatic chemistry, e.g. as described by Pino et al, Lutay et al, Schabarova et al or US 7033753), as depicted in Figure 1 (Step 8) and Figure 2 by the angle arrows, to form the complete sequencible adduct. In this method all the oligonucleotides derived from the target RNA, DNA, etc., that will be sequenced are synthetic, assembled by hybridization and (e.g. enzymatic or non enzymatic) ligation, and prepared for capture onto the sequencing chip by adaptor sequences or (e.g., enzymatic) poly-adenylation. Though the sequencible adduct depicted contains the NPP, one skilled in the art will see that the tag linker containing adduct could instead be prepared as the adduct to be sequenced (prepared as the sequencible adduct), or if not destroyed, the target oligonucleotide could be prepared as a sequencible adduct. Where the target oligonucleotide is sequenced, or comprises the sequencible adduct, then the NPP can consist on non sequencible components, such as LNA's, amino acids, peptides, peptide nucleic acids, aptamers, etc. The sequencible adduct with adaptors at both ends can be prepared such that it is cleaved (e.g. by a second nuclease reaction), providing two sequencible adducts.

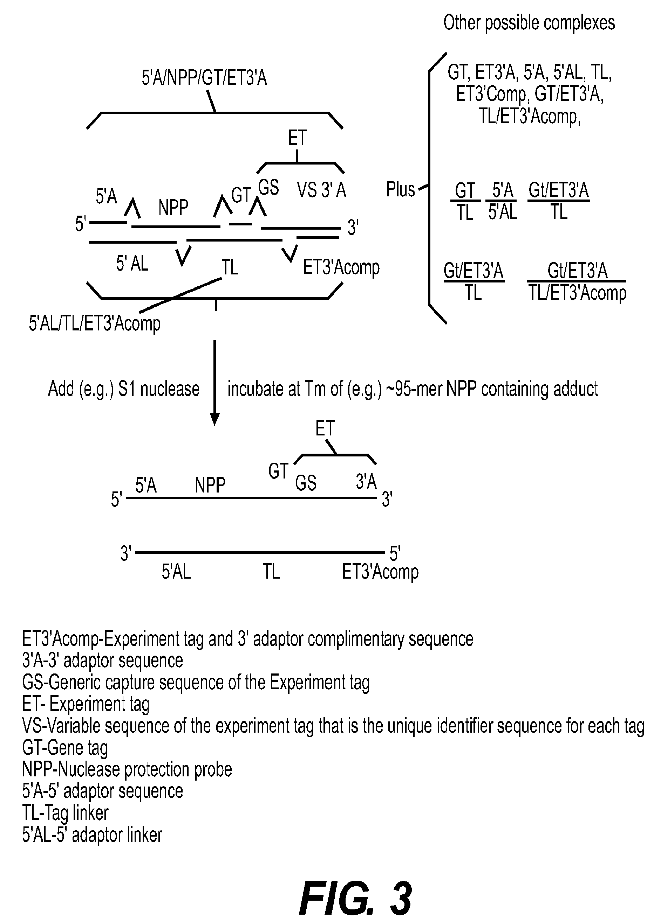

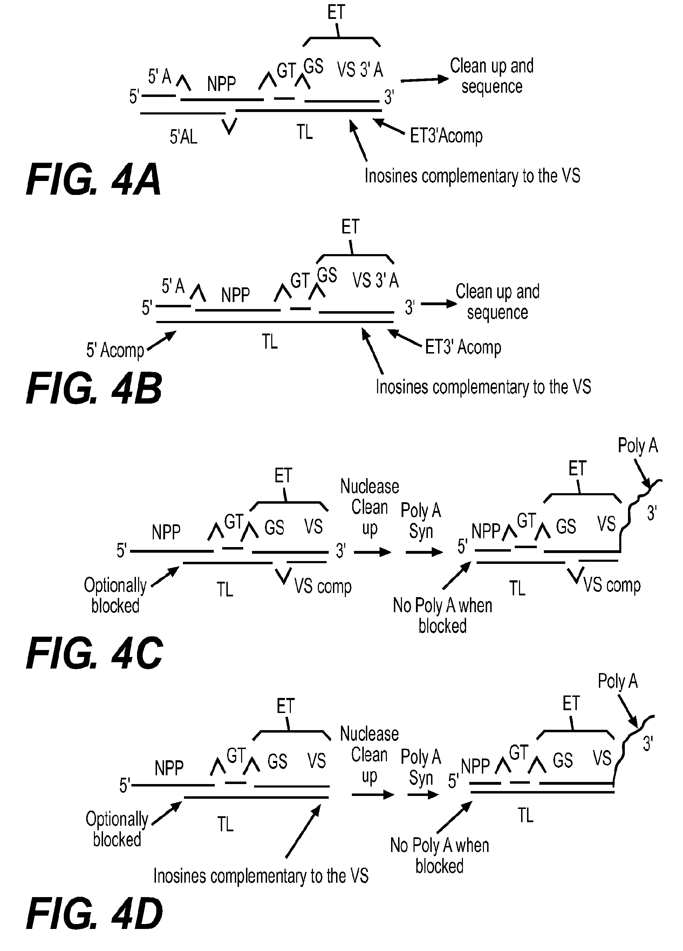

There are numerous ways to attach a poly-A (or Poly-T) capture sequence to the sequencible adduct. One is enzymatically (e.g., using deoxynucleotidyl transferase, Tdt). Another is via hybridization and ligation. A third is simply by synthesis onto the 3' oligonucleotide that terminates the sequencible adduct. Ideally only the sequencible adduct is bound to the sequencing medium, and the side products are eliminated. For example, the adaptor sequence depicted in Figure 3 or 4 can be poly-A, and clean-up can be by gel or nuclease (e.g., S1). In the case of nuclease clean-up the protecting sequence would contain poly-T.

The use of the experiment tag is to differentiate one sample from another. Steps 1 to 5 would be carried out within separate assays for each sample (e.g., separate wells of a microplate), but the tag linker would have been designed to also capture a generic sequence of an experiment tag (see Figure 2C), and the experiment tag (e.g. also containing the 3' adaptor sequence), would be added after step 5, and then steps 6 through 8 carried out, all in separate reaction vessels which demark separate experiments or separate patient samples. One skilled in the art can see that a different tag linker could be synthesized for each experiment tag that contains the complementary sequence to the specific experiment tag sequence rather than a generic sequence added to each experiment tag, shortening the length of the experiment tag to just the experiment specific sequence. After ligation of the experiment tag (or gene tag plus experiment tag) the separate samples can be combined, because the sequence of the experiment tag will identify from which reaction, or from which patient, the sequenced adduct was derived, so in the case of gel purification (or other method of purification or clean up that does not require actual separation) only one gel (or clean up or purification reaction or process) needs to be run per sequencing run.

Ligation with T4 DNA Ligase requires a 5' phosphate to work. Typically oligonucleotides are synthesized without a 5' phosphate, however, the 5' phosphate can be added during synthesis. Thus if the adapter linker and the tag linker are synthesized so that they butt together, but there is no 5' phosphate, they will not be ligated together, facilitating for instance the subsequent clean-up. Another way to add phosphates to oligonucleotides (besides synthesis) is to use T4 polynucleotide kinase and ATP.

Other methods of ligation could be used, including non enzymatic methods. However, ligation is not a requisite step. In the case that the hybridization of the NPP with tag linker and tag, or where a tag incorporated as part of the nuclease protection probe can be protected by a complementary oligonucoeotide, forms a complex that is nuclease resistant or purifiable, no ligation is required because the tag is already incorporated within the NPP and will reflect the amount of NPP, and hence target DNA or RNA, and will identify the NPP, and hence target DNA/RNA when sequenced, even if it is separate from the NPP at the time of sequencing.

All the previous steps represent reagent addition and incubations, no separations until the gel purification or other separation method (if separation is necessary or desired). The excess amounts of each reagent remain present in the reaction mixture (as depicted in Figure 1 to the left of each growing adduct), as well as incomplete adducts such as result from the hybridization of tag linker with the tag molecules but not the nuclease protection probe, or of the adaptor molecules to the adaptor linker but again, in a complex not including the nuclease protection probe. At this point there are several next steps possible, only one of which is depicted (gel purification).

A preferred next step is to clean up the mixture before capture onto the sequencing beads or chip. If the sequences of the adaptor linker and tag linker that hybridize to the nuclease protection probe are separated by several bases (in the case phosphates are added enzymatically post adduct assembly), or they are not phosphorylated (even if they butt up to one another), they will not be ligated together. Then the reaction mixture of all the experiments or patient samples can be pooled together, heated or otherwise denatured to create single stranded oligonucleotides, and the sequencible adduct purified, such as by gel electrophoresis based on its considerably longer length. Other means to effect clean up known in the art or adapted from the art can also be utilized.

Figures 2B through 2E depict preparation of adducts with adaptor sequences. They could instead be prepared without these sequences, but with some other form of capture onto the sequencing chip, or preparation for sequencing. For instance, instead a Poly-A tail could be synthesized onto the 3' end of the sequencible adduct. If it is desired that the complementary strand not be poly-adenylated then the 3' end of that sequence can be blocked, such as by synthesizing the oligonucleotide with a 3'amino residue or with a 3'Carbon (e.g., C3) spacer. This is an advantage of using synthetic sequences to prepare the sequencible adduct, rather than the target itself or a biosynthetic derivative of the target as a part of the sequencible adduct. Some sequencing systems may capture the sequencible adduct directly, such as by a tethered polymerase or oligonucleotide binding moiety, or by chemical or electro or electrochemical means, and thus the sequencible adduct does not require a specific adaptor or capture sequence or moiety.

A preferred method of cleaning up the reaction products for sequencing is to perform a second nuclease digestion, such as again by use of S1 nuclease. In one case an experiment tag/adaptor sequence is added before ligation, and if the adaptor linkers and tag linkers are designed to butt up against one another, with the 5' end of the one phosphorylated, and a complementary 3' experiment tag/adaptor sequence is added such that it can be ligated to the tag linker after hybridizing to the experiment tag/3' adaptor sequence, both the nuclease protection probe containing adduct and the linkers/protecting complementary sequence (respectively) will be ligated together, when the linkers are associated with the nuclease protection probe, forming two complete adducts hybridized to one another (Figure 3A). Treating with S1 nuclease at a temperature that leads to dissociation of all adducts shorter than these two adducts will destroy all the other species (some of which are depicted in Figure 3A), leaving just the sequencible adduct containing the nuclease protection probe, and the linker adduct. Once denatured, only the adduct with the appropriate capture adaptor sequences (the nuclease protection probe adduct) will be captured onto the sequencing chip or beads, and the linker containing adduct will be washed off. The advantage of this "dual S1" approach is that there are no separation steps until the adducts are captured onto the sequencing beads or chip. Figure 4 A depicts a different scheme for forming the sequencible NPP adduct where the tag linker contains inosines at the residues complementary to the experiment tag (ET) variable sequence (VS) (the sequence that when sequenced uniquely identifies the well or experiment), and then the sequence complementary to the 3' adaptor (3'Acomp). This same inosine-containing linker could be used to form the sequencible adducts described above (Figures 1 and 2) and where poly-adenylation is required (rather than use of adaptor sequences), or where gel purification or other separation or purification method is used. Figure 4B depicts the use of a single synthetic combined 5' adaptor tag/tag linker/3'adaptor complement sequence that does not require ligation, and can be made synthetically. In the case of sequencing using a system that does not require amplification, such as the Helicos single molecule sequencing method, a poly-A tail may need to be attached. Figure 4 C and D depicts schemes for this process that can utilize gel purification for clean-up (e.g., prior to poly-adenylation) or as depicted utilize a nuclease step for clean-up before poly-adenylation, capture and sequencing. In both cases, since the NPP itself is not sequenced, only a tag linker is required to hybridize the appropriate gene tag and experiment tag to the NPP so that they can be ligated together. After the nuclease step the poly-A trail is enzymatically synthesized onto the 3' end. This can result in a poly-A tail being synthesized onto the 3' end of the tag linker, such that it too will be sequenced, or if the 3' end of the tag linker is blocked, then the poly-A tail will only be synthesized on to the NPP containing adduct. In the case the NPP is sequenced in its entirety or in part to identify the target gene; the poly A tail or adaptor (if required) can be attached directly to the NPP, or via the experiment tag and/or the tag linker to enable their sequencing. In the case that NPP hybridizes to target (e.g.) DNA, and the system utilizes direct sequencing of the NPP, the NPP-protected (e.g. DNA) target sequence can also be sequenced, and modified for sequencing at the same time and in the same manner as the NPP. Likewise, any complimentary linkers constructed to form a sequencible adduct containing the NPP can be processed in a parallel manner and also be converted into a sequencible adduct. In these instances then two complimentary sequences will be detected, identified, and counted, providing a level of redundancy to the process.

Those skilled in the art can devise other methods for cleaning up the reaction mixture before sequencing, e.g., using gel purification, or biotin/avidin capture and release or capillary electrophoresis or any of a number of separation or clean-up methods. For instance, the nuclease protection probe can be biotinylated or other haptan attached and captured onto a avidin or anti-haptan coated bead or surface, washed, and then released for sequencing. Likewise, the ligated nuclease protection probe adduct can be captured onto an complimentary oligonucleotide, washed and then released for sequencing. The capture oligonucleotides need not be particularly specific, since the qNPS process eliminates most of the genome or transcriptome and leaves just the NPP that had been hybridized to target, and because specificity will be determined at the level of sequencing.

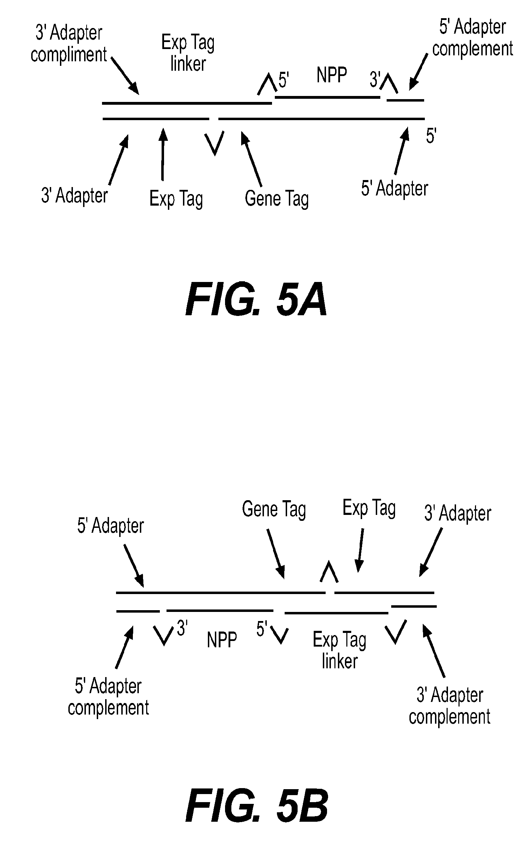

One skilled in the art can also see that the linker complex can be cleaned up and sequenced rather than the adduct containing the nuclease protection probe. Thus the sequencible adduct can be one that hybridizes to the NPP, or is derived from the NPP. Two examples of these adducts are depicted in Figure 5, though others can be configured. Figure 5A depicts use of the same NPP as in previous figures and discussions, but in this case an oligonucleotide is added that contains the 3'adaptor, a complementary sequence to the NPP, and an overhang gene tag sequence that ends in a generic sequence which in turn captures an experiment tag linker. This experiment tag linker in turn captures the experiment tag which also contains the 5'adaptor sequence. If nuclease clean up is to be used, a protecting 5' adaptor sequence probe needs to be added. In the case a poly-A tail is required for sequencing, then the adaptor sequences are not required, and do not have to be included. Figure 5B depicts the use of a nuclease protection probe that is 3' to 5'. This construct can be used for any of the adducts depicted in previous figures or described in previous discussions, and referred to subsequently. The portions of the oligonucleotide (e.g. linker) that hybridize to the NPP can be sequenced to identify the gene, rather than using a gene tag. One skilled in the art will see that there are numerous variations and combinations of and on these arrangements of probes to either result in a adduct for sequencing that contains the NPP or does not.

Sequencible adduct or adducts include or are derived from, or used as a template, a product that survived a nuclease reaction. Sequencible adduct or adducts include or are derived from, or used as a template, a product that survived a nuclease reaction, and is a product from a second nuclease reaction. Sequencible adduct or adduct is a product or derived from a product of one or more nuclease reactions. Synthetic oligonucleotides comprising the sequencible adduct or used to assemble the sequencible adduct can be prepared to permit or not to permit enzymatic or non enzymatic modification, such as ligation or addition of a Poly-A sequence, They can contain natural or unnatural nucleotides (e.g., locked nucleic acids, or LNA's, or peptide nucleic acids, or PNA's, etc.). They can be subject to amplification in solution or on a surface before sequencing, or amplification can be carried out prior to the nuclease protection steps.

For sequencing on the 454 or Solexa platform the sequencible adduct must first be captured and amplified. This typically requires a polymerase reaction. A typical lysis buffer used for qNPS is one designed to denature nucleases to prevent the destruction of RNA, and to facilitate hybridization, while permitting S1 activity. Solutions of this type can inhibit polymerase activity, and thus inhibit the amplification unless the chip is first washed. Washing can also be used to remove nucleotides that do not have the capture adaptor sequence.

In the case where sequencing utilizes a Poly-A tail for capture, this can be synthesized after clean up using terminal deoxynucleotidyl transferase (Tdt), which extends the poly A residues at the 3' end. To prevent the 3' end of the linker containing adduct, or the adduct that is not intended for sequencing, from being extended with a poly-A tail, the 3' residue of the tag linker can be modified with a residue, or modified residue, that does not support poly adenylation (Figure 4C and 4D).

One skilled in the art can see that reverse sequencing can be used with appropriately designed adducts containing the nuclease protection probe and other information containing sequences, or that the complementary sequences to the nuclease protection probe, referred to in some instances as "linkers", and adduct constructs, can be sequenced instead of the nuclease protection probe containing adduct, so long as the complementary adducts are appropriately designed (e.g., see Figure 5), or for instance as described in this application for the nuclease protection probe-containing adducts.

Incubation in (e.g. the qNPA) lysis buffer at 95°C makes RNA accessible for hybridization, though PCR of this lysis product can result in amplification of DNA, demonstrating that there can be genomic DNA in the lysate, just not denatured sufficiently for hybridization of NPP. Incubation at 105°C makes genomic DNA accessible to NPP probe hybridization. S1 (nuclease) processing after 105°C incubation destroys all unhybridized DNA as well as unhybridized RNA and NPP. Because adaptors are hybridized and ligated to the single stranded NPP by use of appropriately designed linker probes with sequences complementary to the 3' or 5' sequence of the NPP, any (e.g., double stranded) DNA (or for that matter RNA) that escapes S1 hydrolysis should not have adpators ligated to them and hence will not be captured onto the sequencing beads or chip used by the 454 and Solexa type sequencers, and will not be sequenced. In the case the NPP complementary oligonucleotides are sequenced, then at least one adaptor can be incorporated directly as a part of the sequence, and hence there is no possibility of that adaptor sequence being ligated to DNA that might have escaped S1 hydrolysis. In the case of gel (or other) purification, the DNA can be separated from the ligated adduct, and thus removed before sequencing. For single molecule sequencing where a Poly-A tail is added to the experiment tag (or to the gene tag in the case no experiment tag is used, or to the NPP in the case no experiment tag or gene tag is used), any DNA may also be poly adenylated unless it is separated first (before poly adenylation) as it would be using gel purification of the sequencing adduct, or destroyed first as for example in the case of using lysis at for example 105°C followed by NPP hybridization and then by a nuclease

(e.g., S1) step under appropriate conditions. In this protocol the NPP can target splice junctions of the mRNA so that no DNA (which could interfere in the measurement of mRNA) will be measured.

miRNA (or siRNA) can also be measured, although in this case the NPP will only be (e.g.) about 22 bases in length to match the miRNA length. DNA and expressed SNP's can be measured, as well as DNA methylation by creating a base mis-match at the site where methylation has or has not occurred, and by judicious use of complementary inosine residues, by the use of additional nucleases or restriction enzymes to cleave the mismatched base residue. Direct sequencing of these adducts, protected by the NPP, is also possible. For instance, a DNA SNP can be sequenced by use of a NPP to the sequence where the SNP may occur, treatment with S1 under conditions that the single base miss-match is not cleaved, and then the surviving DNA target sequence can be dissociated from the NPP by incubating above the Tm of the hybridization, followed by addition of a huge excess of linkers that hybridize to the target DNA and permit appropriate addition of adaptors (the dissociated NPP would be competitively prevented from re-associating by the huge excess of linkers), etc. to create a sequencible adduct that includes the target DNA itself with, as desired, an experiment tag. In a modification of this the NPP could contain an inosine(s) complementary to the SNP site, or multiple SNP or mutated sites within the protected sequence to assure the target DNA is protected during the first nuclease step, and likewise the linker oligonucleotides could contain inosines to assure protection in the case a nuclease clean up step is utilized. Alternatively, NPP probes with the potentially mutated base(s) can be used. In addition, when wild type sequence NPP is cleaved by nuclease at the SNP or mutation mismatch, the particular sequences of the NPP can be processed and sequenced to identify the presence and location of the mutation. In the case that the NPP is used to select a region of target (e.g. DNA) containing mutations under conditions where any mis-matches are not cleaved or hydrolyzed (such as by using an exonuclease, or less stringent conditions with an endonuclease, or by using a nuclease that requires multiple adjacent mismatches for cleavage), then the target (e.g. DNA) can be processed and sequenced to determine precisely the mutation.

It is also possible to incorporate non-target oligonucleotide sequences that can be used as an adaptor to permit capture onto the sequencing chip, or serve as a gene tag or experiment tag directly into the NPP when it is synthesized. This non-target sequence will not hybridize to target oligonucleotide, and normally would be cleaved by nuclease. However, if one hybridizes this non-target sequence of the NPP with a complementary oligonucleotide (either before, at the same time, or after adding the NPP to the sample containing target oligonucleotide, but before the nuclease step), then when treated with nuclease, because every base is hybridized to a complementary base, the non-target NPP sequence will be protected and the NPP will remain intact. Conditions can be modified so that this is true even if there is a single unhybridized base between the nucleic acid target sequence and the non-target sequence of the NPP. This method can produce a directly sequencible NPP adduct, with required adaptor sequence attached, that can be captured on the sequencing chip and sequenced without use of any ligation reaction. Those familiar in the art can design methods to clean up the reaction before sequencing to remove the short non-target sequence/complementary sequence duplexes. For instance, one can heat up the post nuclease sample in base to dissociate the duplexes, then add an excess of an oligonucleotide that is complementary to the non-target sequence of the NPP and a portion (e.g. the first 25 bases) of the nucleic acid target-specific sequence. If hybridization is then carried out at a temperature where this longer oligonucleotide can hybridize but not the shorter non-target sequence complementary oligonucleotide, a preparation is obtained which after a second nuclease reaction will only contain the NPP that had been hybridized to nucleic acid target. This can then be heated to cause its dissociation and then added to a sequencing chip where it can be captured through its adaptor sequence and sequenced.

In the case increased sensitivity is desired, the target oligonucleotide or a product derived from it can be amplified, or the NPP product can first be subject to PCR or other forms of enzymatic amplification. The resulting product can then be prepared for sequencing in the same manner as the unamplified NPP product, or during the process of amplification the gene tag and/or experiment tag, and/or adaptor sequences can be incorporated as, for instance, part of the primer and extension constructs. Even when amplification is not required, one or two cycles of PCR or enzymatic reaction can be carried out to attach a gene tag, and/or an experiment tag, and/or the adaptors. This adduct generated from the NPP by subsequent biosynthetic step or steps, can also be completed by hybridization reactions such as those described for generating the sequencible NPP adducts or adducts complementary to the NPP. Clean up can be via gel or other purification method, or with sufficient protection, by a subsequent S1 (or other nuclease) reaction or other means known in the art or adapted from the art.

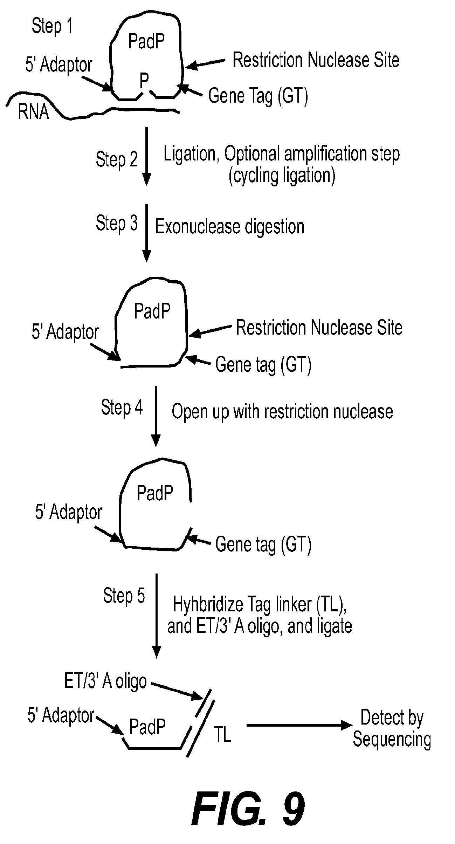

Another type of NPP is a circular probe, similar to Padlock (PadP) or circular DNA probes (e.g. similar to the constructs described by Baner et al or Prins et al). PadP sequencible adducts are depicted in Fig 9. This PadP construct can be constructed to contain adaptors and tags, which will not be cleaved when an (e.g.) exonuclease is used after hybridization of probe to target in the sample. For instance, the PadP probes can be synthesized to contain the 5'adaptor, and about 10 to about 30 or about 50 or about 100 or about 200 bases at its 3' end that hybridize to the target. There can be a spacer region, then a restriction nuclease site, then a 5' gene tag, then the rest of the PadP probe that hybridizes to the target (another about 10, or about 30, or about 50 or about 100 or about 200 bases), phosphorylated at its 5' end to support ligation. Thus, when hybridized to target (Step 1) the two halves of the PadP probe can be ligated to form a circular DNA adduct. By cycling this can be amplified (Step 2). After ligation the mix can be heated to about 95°C to dissociate the circular probe from the target (e.g. RNA), then temperature is lowered so excess probe can rehybridize to the target (e.g. RNA) which serves in this case as an amplification template, then after ligation the temperature is raised again, for a series of about 30 cycles to produce about 30 copies of circular probe/target template RNA. In Step, 3 exonuclease is used to destroy all linear DNA (and e.g. target RNA), including excess PadP, leaving only the circular PadP probes. Step 4 begins the process of tagging with the experiment tag if desired, first treating with restriction enzyme to open up the circular DNA probe, then using a tag linker to hybridize and ligate the experiment tag. Experimental conditions used to form the PadP probes have been described.

NPP constructs can be designed that can be directly sequenced, a method referred to as "direct nuclease probe sequencing" (DNPS). One such construct is depicted in Figure 1. In the case where the nuclease protection probe is directly sequenced and current commercial methods of adding adaptor sequences for sequencing or adding a poly-A tail or other capture molecule is used, the S1 product can be directly sequenced. However, where adducts are ligated together by use of linkers, be it due to the addition of an adaptor, a gene tag, an experiment tag, or other sequence, the excess tag probes, adaptors, or linkers, may need to be eliminated in a "clean up" step before sequencing. Several strategies can be used. The simplest strategy is to incubate at a temperature below the melting temperature (Tm) of the ligated adduct that will be sequenced (e.g., the complex of probe, detection linker, experiment tag, gene tag and as needed adaptors), but above the melting temperature for the linkers and linkers complexed to components of the adduct, but not the complete adduct itself. In this way, they melt apart and, along with unhybridized linkers, experiment and gene tags, are destroyed by S1 (or other nuclease or cocktail of nucleases). The (e.g.) S1 activity is then destroyed, such as by heating to 95°C or enzymatically by use of proteinase K or by use of an inhibitor. This "two stage" nuclease protection approach results in a protocol that is an add-only process without any separation steps, up to the point of capture onto the sequencing surface.

Sequencing of genes and determination of abundance by sequencing of nuclease protection probes can be carried out without sequencing the entire nuclease protection probe. If the 3' end of the nuclease protection probe is selected so that the combination of the terminal 2 to about 7 or about 25 bases represent a unique sequence for each gene measured, then this is all of the nuclease protection probe that needs to be sequenced to identify the gene, and by counting the number of such adducts sequenced, the amount of each gene in the sample. Experiment tags (a different one for each experiment) can be appended to the nuclease protection probe to permit the qNPA products of multiple experiments to be pooled together for sequencing.

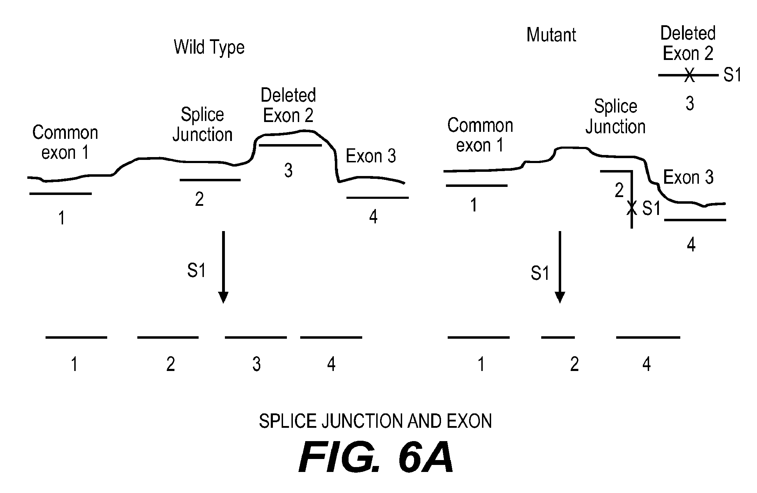

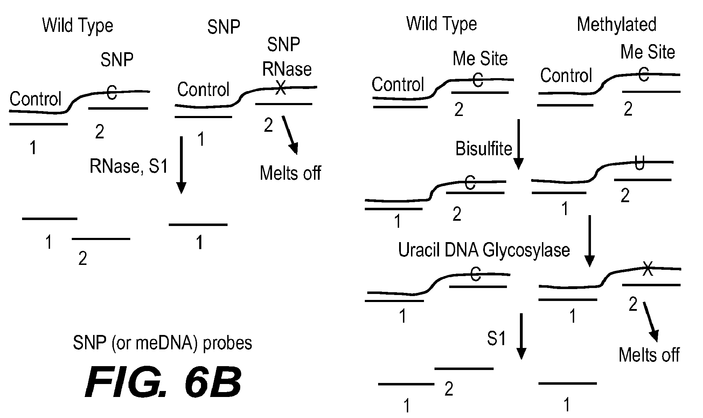

Examples of how splice junctions, exons, and mutations can be sequenced and quantified, and the result after completing the nuclease protection steps are depicted in Figure 6A. Examples of how single nucleotide polymorphisms (SNP's) and methylated DNA can be sequenced are depicted in Figure 6B, These single base modifications are detected by utilizing the activity of additional enzymes such as RNase to detect expressed SNP's, or the combined effects of bisulfite treatment followed by uracil DNA glycosylase to detect methylated DNA sites. One skilled in the art can see how DNA SNP's could similarly be detected and measured by sequencing. In each case a control sequence, common to the target gene or all variants of the target gene, is designed, together with probes specific for the (potentially mutated or methylated) site of interest. Probes can also be designed to hybridize to a specific splice junction, a specific exon that may be deleted or a specific gene fusion. The red "x"s indicate probe sequences that are not protected and therefore degraded by, for example, S1 or where the target sequence will be cleaved and therefore the nuclease protection probe will melt off and be destroyed by S1. In the case of only a single mis-matched base, it may be necessary to add an additional enzyme or enzymes to e.g., S1 such as RNase, or to use a different enzyme that cleaves the single base. Those skilled in the art will see that there are numerous enzymes, modified enzymes, or molecules with similar activity that could be used alone or in combinations to perform these cleavages. The nuclease protection probes can be further modified by the addition of experiment tags (using the methods described elsewhere in this invention) to permit samples from multiple experiments to be combined into a single sequencing run. The sequencing adaptor sequences can be ligated onto the nuclease protection probe (or in the case an experiment tag is used, also to the experiment tag, if the experiment tag was not itself synthesized with the adaptor sequence at its 3' end). The 5' end of the nuclease protection probe may be phosphorylated during its synthesis, then a linker used which hybridizes to the 5' bases (e.g., 25 bases) of the nuclease protection probe and has a complementary sequence which hybridizes to the 5' adaptor sequence, thus appending the adaptor to the 5' end of the nuclease protection probe where it can be ligated together (e.g., using T4 DNA ligase). Alternatively, addition of ATP and use of an appropriate DNA ligase (e.g., T4 DNA ligase) can self-phosphorylate and ligate. For the 3' adaptor, the adaptor itself can be phosphorylated, and the linker designed to hybridized to the 3' bases (e.g., 25 bases) of the nuclease protection probe and to contain a complementary sequence to the 3' adaptor, such that it hybridizes and is apposed to the 3' end of the nuclease protection probe in a manner that permits it be ligated onto the probe. Under appropriate conditions the 5' ends can instead be phosphorylated using T4 polynucleotide kinase and ATP, then ligated using T4 DNA ligase. Under other appropriate conditions T4 DNA ligase can itself phosphorylate and then ligate. In the case that a Poly A tail needs to be added to the 3' end of the nuclease protection probe, it can be added using Tdt.

In a preferred method there is one (or more) nuclease protection probe that measures a sequence of the target gene that is homologous between wild type and mutant, or which does not undergo methylation in the case DNA methylation is being measured, and then a second probe designed against the site of the mutation or DNA methylation. Thus the total level can be determined as well as the proportion of mutation.

qNPS can also be used to detect unknown mutations simply by making probes against various regions of the target gene and then sequencing the probes from the qNPA reaction. The probes can be incorporated into constructs that include experiment tags, and adapter sequences can be incorporated into the adduct for sequencing. Advantage can be taken of nuclease activity of one or a combination of enzymes to cleave bases that are mis-matched, and as desired to detect SNP's. In the case those bases are located toward the end of the nuclease protection probe then at the temperature of cleavage the entire short strand will melt away and be destroyed, leaving a shortened probe sequence. If toward the middle of the probe, then conditions can be routinely designed such that all sequences will melt apart and be destroyed. Alternatively, if an SNP or several mis-matched bases are located within the middle region of the nuclease protection probe, conditions can be used where the nuclease protection probe is cleaved but does not melt off, and then sequencing will identify the specific mutation site. By using multiple probes against the same gene, the probe counts can be compared to identify where mutations occur. In this scenario the ligation of the required adapters can be carried out in the manner used today for sequencing on the respective platforms. The sequence of the nuclease protection probe ends remaining will not be known, and thus adapter linker sequences cannot be designed. Alternatively, adaptors with nuclease protection probe end hybridizing inosine sequences can be used - where the specific composition of the ends of the nuclease protection probe does not have to be known. Alternatively, the adapter modification process can be carried out as described elsewhere. The adaptors would be ligated properly to intact NPP, and hence only these would be sequenced.

In all the examples given the adaptor sequences, poly-A sequence, or other required capture molecule(s), if required at all, can be added to the NPP or adduct with gene tags or experiment tags using methods known in the art or practiced for sequencing without use of the linkers and process described in various instances in these examples.

For single molecule sequencers either the nuclease protection probe, with or without experiment and gene tags, or the probe with a 3' capture sequence attached can be sequenced without the need for adaptor sequences at all, or with only the adaptor (or capture) sequence at the 3' end. For attachment of experiment identifier and gene identifier tags a ligation step may be necessary (e.g., using T4DNA ligase), followed by clean up, and then as necessary (e.g., for next generation sequencers such as Helicos), attachment of only one adapter sequence (e.g., at the 3' end), or attachment or synthesis of a poly A tail, (e.g.,) extension at the 3' end of a poly A tail using (e.g.,) Terminal deoxynucleotidyl transferase (Tdt), or attachment of another universal capture sequence or molecule is required to permit capture onto the sequencing chip. Constructs described here and elsewhere in this instant invention can all be prepared for sequencing on such instrumentation. Figures 2, 4, and 5 depict constructs designed for multiplexing experiments within the same run/channel of the sequencer, and for using gene identifier tags to reduce the read length required. For attachment of tags a ligation step is necessary (e.g., using T4DNA lygase) after the nuclease protection steps 3 to 5 have been carried out, followed by clean up, and then as necessary (e.g., for next generation sequencers such as Helicos), extension at the 3' end of a poly A tail using Terminal deoxynucleotidyl transferase (Tdt) to permit capture onto the sequencing chip, or an appropriate adaptor molecule. Note that if the lysis buffer inhibits any of these steps, then a dilution buffer which permits reverse transcription and PCR can be used. The detection linker of the array-based assay is used to link an experiment tag to the nuclease protection probe. Then the probe and the tag are ligated together using T4 DNA ligase. A gene identifier tag can also be incorporated, potentially reducing the sequence read to 10 bases (just the two tags). Alternatively, by selecting target gene sequence regions so that the 3' ends of the nuclease protection probes are unique for each gene (e.g., the sequence of five to seven of the 3' terminal bases are unique), only this region must be sequenced to identify each gene measured.

Tags that are not complementary to target DNA or RNA can be directly incorporated into the NPP (e.g. by synthesis) and protected by a complementary oligonucleotide sequence during the nuclease step so it will not be hydrolyzed, or it can be composed of a sequence that is resistant to hydrolysis by nuclease yet still sequencible. By the tag sequencing oligonucleotide butting up to the target sequence, nuclease cleavage can be prevented so long as there are no unpaired bases in the NPP construct.

Advantages of performing the detecting step of qNPA assays by sequencing include: sequencing identities without extraction, e.g., from solid phases such as tissue; avoidance of the need for separate detection operations for each of multiple samples - all can be performed in one solution simultaneously; avoidance of weak cross-reactivity among probes, e.g., due to use of high concentration of detection linkers; enhanced SNP determinations; etc.

In one embodiment, the present disclosure provides for the following aspects:
Aspect 1: Sequencible adduct or adducts do not contain the target oligonucleotide.
Aspect 2: Sequencible adduct or adducts do not contain the target oligonucleotide, nor were formed using a biosynthetic step.
Aspect 3: Sequencible adduct or adducts include or are derived from, or used as a template of, a product that survived a nuclease reaction.
Aspect 4: Sequencible adduct or adducts include or are derived from, or used as a template of, a product that survived a nuclease reaction, and is a product from a second nuclease reaction.
Aspect 5: Sequencible adduct or adducts are a product or derived from a product of one or more nuclease reactions.
Aspect 6: Sequencible adduct or adducts form through use of synthetic oligonucleotides.
Aspect 7: Sequencible adduct or adducts form through use of synthetic oligonucleotides and hybridization reactions.
Aspect 8: Sequencible adduct as in 7, further formed from the use of ligation reaction. comprising the sequencible adduct or used to assemble the sequencible adduct.
Aspect 9: Synthetic oligonucleotides comprising the sequencible adduct or used to assemble the sequencible adduct, assembled based on, or incorporating, a NPP.
Aspect 10: Synthetic oligonucleotides comprising the sequencible adduct or used to assemble the sequencible adduct, prepared to permit or not to permit enzymatic modification, such as ligation or addition of a Poly-A sequence, and containing or not containing unnatural nucleotides (e.g., locked nucleic acids or peptide nucleic acids, etc.).
Aspect 11: Sequencible adducts containing or assembled based on a NPP subject to amplification in solution or on a surface before sequencing.
Aspect 12: Sequencible adduct or adducts that contain a sequence that is attached subsequent to producing an amount of sequencible adduct that quantitatively reflects the amount of target oligonucleotide which sequence (e.g., gene tag), can be used to identify the adduct and hence the target oligonucleotide.
Aspect 13: Sequencible adduct or adducts that contain a sequence that is attached subsequent to producing an amount of sequencible adduct that quantitatively reflects the amount of target oligonucleotide, (which sequence e.g., experiment tag) can be used to identify the reaction containing the target oligonucleotide, and hence permits multiple reactions to be pooled and sequenced at the same time.

### BRIEF DESCRIPTION OF THE DRAWINGS

Various features and attendant advantages of the present invention will be more fully appreciated as the same becomes better understood when considered in conjunction with the accompanying drawings, in which like reference characters designate the same or similar parts throughout the several views, and wherein:

Figure 1 provides a schematic outline of the production of the nuclease protection probe (NPP) from sample for the quantitative nuclease protection sequencing (qNPS) assay. The use of a linker (green) to attach a gene (or experiment) tag with any required acceptor sequence (blue) is depicted, as well as the use of a separate linker (purple) to add an adaptor (red) to the other end of the NPP, followed finally by ligation. In each case not only is the sequencible adduct formed, but excess linkers, adaptor and tag sequences accumulate. The use of gel purification to separate the sequencible adduct from the other short sequences is depicted.

Figure 2 depicts the processing of NPP for sequencing. Figure 2A depicts two possibilities in subsequent processing, involving poly-A addition or adapter sequence addition. Figures 2B through 2E depict preparation of adducts with gene tag, experiment tag, and or adaptor sequences.

Figure 3 outlines qNPS probes and tagging adducts that are useful for sequencing and use of a nuclease step for clean-up. The bent arrows indicate points of ligation. The sequencible NPP adduct and its complement are generated. The key defines the different oligonucleotides used to form the sequencible adducts.

Figure 4 depicts an alternative method of assembly of the sequencible adduct. Figure 4A depicts a different scheme for forming the sequencible NPP adduct where the tag linker contains inosines at the residues complementary to the experiment tag (ET) variable sequence (VS) (the sequence that when sequenced uniquely identifies the well or experiment), and then the sequence complementary to the 3' adaptor (3'Acomp). Figure 4B depicts the use of a single synthetic combined 5' adaptor tag/tag linker/3'acaptor complement sequence that does not require ligation, and can be made synthetically. Figures 4C and 4D depicts schemes for this process that can utilize gel purification for clean-up (e.g., prior to poly-adenylation) or as depicted utilize a nuclease step for clean-up before poly-adenylation, capture and sequencing.

Figure 5 depicts sequencible adducts that contain and do not contain the NPP.

Figure 6 provides a depiction of how splice junctions, exons, and mutations can be sequenced and quantified. Figure 6A Legend. Probes for measuring mRNA at a region that is common to all variants of a gene. (Common exon 1) and for measuring a splice junction between two exons (exon ½ junction) where the junction can be exon 1 to exon 2 or exon 1 to exon 3, or for measuring exons (2 and 3), one of which might be deleted (exon 2) are depicted for wild type (left) and mutant where exon 2 is deleted. Note that the deletion of exon 2 results in the total destruction by S1 of the probes for exon 2, and destruction of the exon 2-specific half of the probe for the exon ½ splice junction, indicated by the red "x" S1. Examples of how single nucleotide polymorphisms (SNP's) and methylated DNA can be sequenced are depicted in Figure 6B. Figure 6B Legend. Probes for measuring an expressed SNP (left panel) or a methylated DNA site (right panel) are depicted. In the case of expressed SNP, two possibilities are depicted, wild type or SNP. Two probes are used, one for a control region (1), one with the SNP located in the middle of the probe (2). Treatment with Rnase cleaves the mismatch in the SNP, and then the probe (now just 25 bases each end) melts off at the 50-mer Tm used for the S1 reaction, and is destroyed by S1. For methylated DNA, the same two probe strategy is used, but first bisulfite is used to convert unmethylated C to U, creating a mismatch, and then uracil DNA glycosylase is used to cleave the DNA, so that the probe will melt off and be destroyed by S1.

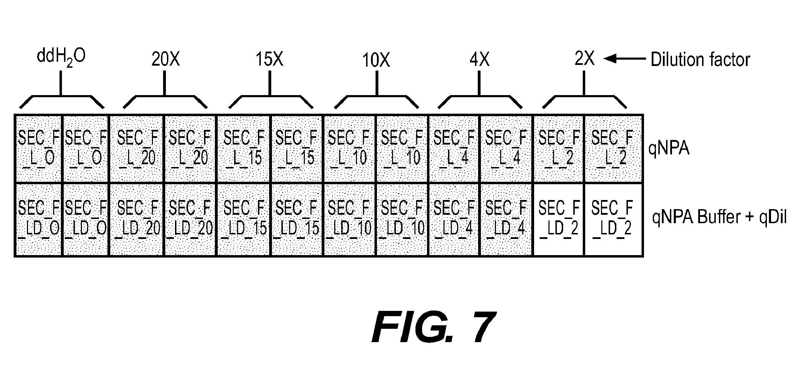

Figure 7 depicts the successful sequencing of a transcript spiked into the lysis and hybridization buffer solution that is produced at the end of the qNPA process. Figure 7 Legend. Sanger sequencing method, ABI 3700, was used. Linear DNA samples (-2.5 kb PCR) with required primers (T7F) for sequencing were submitted to the University of Arizona core sequencing facility. qNPA lysis buffer with or without addition of a dilution buffer (qDil) was diluted from 2X to 20X. qDil was added 1:1, causing a 2X dilution. Each dilution is repeated twice. Same dilutions were also repeated with reverse primer (results not shown). For sequencing, 2.5 µl of 50ng/µl of DNA was mixed in a total volume of 15 µl of reaction mixture. This accounts for 6X dilution. Red, no sequence, light green 50-100 bp sequences, dark green 500-600 bp sequence.

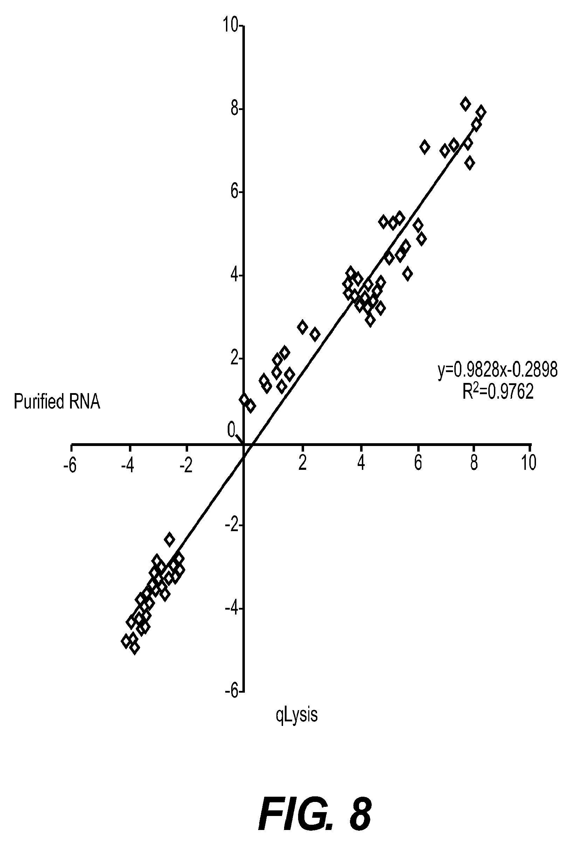

Figures 8 depicts PCR results measuring matched lysates versus extracted RNA to demonstrate equivalence of CT values. Herein, PCR of RNA purified from samples or the qLysis product from the same samples was carried out to measure three genes plus the housekeeper gene GAPDH across a large set of different cell sample mixtures. Each data point is the average of three replicates. Each mixture was tested in three different experiments. The CT values were normalized by subtracting the CT value for GAPDH. The purified RNA was adjusted for the dilution factor required for the qLysis samples and shows the sequence of steps required to generate the PadP sequencible probes. The gene tag and 5' adaptor are part of the original PadP probe, along with a restriction site. The probe is ligated across the target RNA to form circular DNA, and then this is opened up and the experiment tag and 3' adaptor is hybridized and ligated, preparing an adduct for sequencing.

Figure 9 shows a representative schematic method for the generation of PadP sequencible adducts. The gene tag and 5' adaptor are part of the original linear probe, along with a restriction site. The probe hybridizes to the target nucleic acid in such a way that the 5' and 3' ends of the probe are hybridized to adjacent bases, and thus can be ligated together on the nucleic acid template to form a circular (e.g. DNA) probe. Then a nuclease (e.g. an exonuclease) is added to destroy the unhybridized nucleic acid target and excess linear probe. Then the probe is separated from the nucleic acid target (e.g. with heating in base), and the nuclease activity is destroyed. The circular probe is opened up and, as desired, the experiment tag and, as required, 3' adaptor is hybridized and ligated, preparing and adduct for sequencing. The process of hybridizing linear probe to the nucleic acid target, ligation to form circular probe, and dissociation from the nucleic acid target can be repeated in multiple cycles by cycling heating to cause dissociation. Because of the excess of linear probe when the temperature drops linear probes will hybridize, which in turn can be ligated and then released upon the next cycle of high temperature, thus amplifying the amount of circular probe before carrying out the nuclease hydrolysis step.

Without further elaboration, it is believed that one skilled in the art can, using the preceding description, utilize the following invention to its fullest extent. The following specific preferred embodiments are, therefore, to be construed as merely illustrative, and not limitative of the remainder of the disclosure in any way whatsoever.

### Example 1:

The lysis buffer used for the qNPA assay is designed to inactivate enzymes and prevent the degradation of RNA, but after a limited dilution into a hybridization dilution buffer it permits S1 activity and facilitates hybridization with stringent specificity. However, the lysis buffer components inhibit reverse transcription and polymerase activity. Inhibition of polymerase activity thus can prevent successful PCR unless the buffer is removed or the inhibitory activity is diluted out or the inhibitory activity is neutralized. A dilution buffer can be added after the nuclease assay is complete to neutralize the inhibitory activity of the lysis and other buffers. Figure 7 depicts the successful sequencing of a transcript spiked into the lysis and hybridization buffer solution that is produced at the end of the qNPA process, A 10-fold dilution into water permits sequencing to be successful. However, if a neutralizing dilution buffer (qDil) is used for dilution rather than water, then only a 4-fold dilution is required to produce the same sequencing result as for the transcript sequenced out of water (read lengths of 500 to 600 base pairs). However, use of the neutralizing qDil dilution buffer permitted sequencing after just a 2-fold dilution, though the read length was reduced to 50 to 100 base pairs, and therefore was successful but impacted by the lysis and hybridization buffers. Recognizing that for systems where PCR of the target DNA is required before sequencing, there may also be interference from the lysis and hybridization buffers, we tested the efficiency of PCR using cDNA prepared from cells versus lysates prepared from the same cells and diluted with the qDil dilution buffer. There was no difference across mixtures, measuring three genes normalized to GAPDH. (Figure 8). The correlation was 0.97.

### Example 2:

NPPs were designed specific for splice junctions or exons, as well as other regions of target genes, so that in each case the probe is specific for a sequence found only in a single gene in the transcriptome. To permit direct sequencing (direct nuclease protection probe sequencing, or DNPS) of the nuclease protection probe, or a portion of the probe, ideally the first five, ten, twenty, or thirty 3' bases are sufficiently specific that their sequencing uniquely identified just one gene. After the nuclease reaction the remaining probes are prepared for sequencing by incorporating them into sequencing adducts containing the required adaptor or capture sequences or molecules as described previously and below. In an alternative method experiment tags are added to the 3'end. In yet another method, gene tags are added to the 3' end so that the nuclease protection probe sequence itself does not have to be sequenced, nor does the 3' end of the probes have to be specific for only one gene in the transcriptome. In yet another protocol both gene tags and experiment tags are incorporated into the adduct to be sequenced. In yet another example the complementary sequence to the NPP is prepared and the sequencible adduct by methods described previously and below..

### Example 3:

Construction of NPP containing adducts with gene tags and experiment tags. An advantage of this method is that the tag hybridization steps follow the S1 and base steps, where all the native (e.g., RNA) is destroyed, so specificity need only assure that the correct tag hybridizes to its own complement and not to the complement of another tag. Similarly, only the nuclease protection probers need to be target specific. The probes are not themselves sequenced. Instead, a gene tag is incorporated into the adduct which is the entity that is sequenced to identify the gene measured by that specific nuclease protection probe to which then gene tag specifically hybridizes. Following a standard protocol for performing qNPA (3,4) on FFPE, samples are lysed in lysis buffer, with the addition of proteinase k in the presence of a cocktail of nuclease protection probes. After an initial incubation for 30 min at 37oC the sample is heated to 95oC, then cooled and incubated at 55oC for 2 hr to permit the probes to hybridize to their respective target mRNA. Then S1 nuclease is added to hydrolyze excess probes not hybridized to target, and RNA not hybridized to probes, leaving the target/probe duplexes. After a 60 min incubation, base is added and the sample heated to 95oC for 10 min, dissociating the probe/RNA duplexes and hydrolyzing the target RNA sequences. The sample is neutralized, and then a cocktail of 3'tag linkers is added, each with a specific 25 base sequence complementary to the 3' 25 base sequence of one specific probe, and containing a sequence specific to one gene identifier tag. In a second instance the tag linker also contained a sequence 3' to the gene tag sequence which is generic, specifically hybridizing to a 5' terminal sequence common to a set of experiment tags. The gene tag sequence can consist of a number of designs, but in this instance consists of sequence that was complementary to a 5' terminal sequence of the gene tag that is not sequenced, and then a 7-base tag sequence that is unique for each gene tag, and is the 3' terminal sequence of each gene tag that is sequenced to identify each gene. In the case where the 3' terminal sequence of the tag linker also hybridizes to an experiment tag, the 5' complementary sequence of the experiment tag is the same for every experiment tag. Since each different experiment tag is added to separate individual experimental nuclease protection reactions (e.g., separately assayed samples), there is no possibility of the "wrong" experiment tag hybridizing. In this case each sample is prepared in a separate well of a microplate, and a different experiment tag is added to each well. Though additions of tag linker, gene tag and experiment tag can be sequential, in this example all are added together, the tag linker being added in excess relative to the nuclease protection probes surviving the S1 nuclease protection reaction, but at a limiting concentration relative to the amount of gene tag added and experiment tag added so that all the tag linker is saturated with the tag sequences themselves. The gene tags and the experiment tags are all phosphorylated at their 5' end. In addition the experiment tag contains an adaptor sequence at its 3' end complementary to the 3' capture sequence on the Solexa sequencing chip. The 5' end of the nuclease protection probes are also phosphorylated. At the same time that the 3' tag linker and tags are added, a cocktail of 5' adaptor linkers is added, comprised of sequences which contained a gene-sequence complementary to the 5' end of each probe, and a 5' sequence complementary to the 5' adaptor sequence that is captured by the 5' capture sequence of the Solexa sequencing chip. The 5' adaptor sequence itself is added at the same time, in excess of the 5' adaptor linker. Following incubation at 50oC for all the appropriate hybridizations to occur, forming the adduct depicted in Figure 2Exx, a ligation reaction (using T4 DNA ligase) is then carried out. The reaction mixture is subsequently run on a gel and the high molecular weight band cut out and applied to the Solexa chip, amplified and sequenced. In this example the gene tag consists of two identical gene identifying sequences, providing sequencing redundancy for the identification of each gene. In addition, the 5' end of the experiment tag, used for hybridization to the tag linker, contains LNA's at every other position, providing a higher Tm for the number of bases in this sequence, and keeping it as short as possible so that the read length required to sequence the experiment tag and the gene tag was is short as possible.

### Example 4:

The same process described in Example 3 is carried out, except that gel purification is not used. Instead, a 5' phosphorylated adaptor linker and a 5' phosphorylated tag linker is used, and an oligonucleotide is added to each reaction that is complementary to the experiment tag added to that reaction and the 3' adaptor sequence, as depicted in Figure 3. Thus, when the ligation step is carried out this short oligonucleotide is ligated to the tag linker, so that in the complete hybridized and ligated adduct there are no sequences shorter than 100 bases. The reaction mixture is then incubated with S1 nuclease at 65 °C as a "clean-up" reaction. At this incubation temperature the Tm of the components of the complete adduct is such that no parts melt off and are hydrolyzed by the S1, while the hybridizations between excess tag linker and each tag (since the ligated tags are less than 50 bases) and the excess 5' adapter and adaptor linker melt sufficiently that S1 hydrolyzes them. The adduct surviving this S1 reaction is then heated to 95 °C to melt it away from the protecting linker and destroy the S1 activity, and then the adduct is captured on the Solexa chip, amplified, and sequenced.

### Example 5:

An example of constructing adducts for sequencing on a system that utilizes a Poly-A tail to capture the sequencible adduct on the sequencing medium, e.g., for a Helicose system, is carried out. The adduct depicted in Figure 2C is constructed, without the 3' adaptor. Subsequent to gel purification clean-up this adduct is poly-adenylated at its 3' end using Tdt. This same adduct is prepared and cleaned up by S1 nuclease before the Tdt reaction and then sequenced. Alternatively, the adduct depicted in Figure 2C is constructed, with a Poly-A 3' adaptor synthesized as part using Tdt the experiment tag, and this product is cleaned up by gel purification before sequencing. Using a poly-T complementary sequence to protect the poly-A tail, S1 nuclease is employed to clean-up the adduct before sequencing.

### Example 6:

The experiment of Example 4 is carried out using whole blood as the sample. The whole blood is mixed 1:1 with 2x (double concentration) lysis buffer, heated to 95 °C for 10 min, then centrifuged in a microfuge to remove clumps. The supernatant is then subjected to qNPS as described in Example 4.

### Example 7:

The experiment of Example 4 is carried out using a sample of human cells infected with virus. The probes used are designed to measure the viral genes. The results demonstrate the ability to selectively measure the viral genes in the background of human genes, as an example of measuring the genes from any species within a mixture of other species without interference or "cluttering" of the sequenced samples by unwanted sequence information.

### Example 8:

The experiment of Example 4 is carried out using a series of samples consisting of mixtures of lysates from undifferentiated Thp-1 cells and differentiated and LPS stimulated Thp-1 cells.

### Example 9:

Samples are lysed and incubated at 95°C, followed by hybridization with NPP, treatment with S1, addition of tag linker, gene tags, experiment tags, hybridization and ligation, and then are incubated at 105°C, followed by addition of an experiment tag protecting sequence containing LNA's, incubation at 37 °C to permit re-hybridization of the ligated adduct complementary oligonucleotide sequences of 20 bases or more (excess tag linker, gene tags, and experiment tags and experiment tag protection sequence will still be present), followed by S1 hydrolysis and then polyadnylation, and finally clean up by gel electrophoresis and then sequencing. Only one copy of the complementary DNA (to which the tag linker can hybridize) is sequenced, and does not contain the experiment or gene tags. So if 100 genes are measured, there are only 100 molecules/cell of this complimentary DNA sequenced as background, and these sequences do not contain any gene tag or experiment tag sequence information.

### Example 10:

NPP are synthesized that contain, besides the sequence of bases complementary to the target nucleic acid, a non-target sequence that can serve as a capture adaptor sequence for capture onto the sequencing chip, or a sequence that can serve as a gene tag" or a sequence that can serve as an experiment tag, or a sequence that incorporates several of these functions. The NPP is combined with an excess of oligonucleotide that is complementary to the non-target sequence of the NPP and incubated so that they can hybridize together. Then this mixture is added to sample containing target nucleic acid, and after hybridization, is treated with S1 nuclease, carrying out the standard qNPA protocol. Because there are no bases which do not have a complementary based hybridized to them between the portion of the NPP hybridized to the nucleic acid target and the portion hybridized to the non-target complementary oligonucleotide the NPP hybridized to the nucleic acid target is not cleaved by S1 nuclease, but rather remains intact which NPP that is not hybridized to target oligonucleotide is hydrolyzed up to the point of the protected non-target sequence. After heating in base a complementary oligonucleotide is added that spans both the non-target sequence and a portion of the target oligonucleotide sequence, and permitted to competitively hybridize to the NPP at a temperature where only the NPP containing complementary nuclease target sequence will hybridize, and neither the shorter non-target sequence protecting oligonucleotide nor surviving non-sequence NPP sequence fragment can hybridize. Then a second S1 nuclease treatment is performed, and then the surviving NPP, which has the sequence required for capture onto the sequencing chip, can be sequenced. This protocol does not require any ligation to attach the adaptor sequence, since it is part of the synthetic NPP adduct.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the scope of the claims, can make various changes and modifications of the invention to adapt it to various usages and conditions.

### References:

1. Martel, R.R., I.W. Botros, M.R Rounseville, J.P. Hinton, R.R. Staples, D.A. Morales, J.B. Farmer, and B.E. Seligmann. Multiplexed screening assay for mRNA combining nuclease protection with luminescent array detection. Assay and Drug Development Technologies. 2002, 1 (1-1):61-71.
2. Martel. R., M.P. Rounseville, I.W. Botros, R. Kris, S. Felder and B.E. Seligmann. Multiplexed Molecular Profiling (MMP) Transcription Assay in ArayPlates for High-Throughput Measurement of Gene Expression in Gene Cloning and Expression Technologies, Q. Lu and M. Weiner, Eds., Eaton Publishing, Natick (2002).
3. Robin Roberts, Costi Sabalos, Ralph Martel, Michael LeBlanc, Joseph Unger, Ihab Botros, Bruce Seligmann, Thomas Miller, Thomas Grogan and Lisa Rimsza (2007) "Quantitative Nuclease Protection Assay in Paraffin-Embedded Tissue Replicates Prognostic Microarray Gene Expression in Diffuse Large-B-Cell Lymphoma" Laboratory Investigation, 87: 979-997.
4. Lisa Rimsza, Michael LeBlanc, Joseph Unger, Thomas Miller, Thomas Grogan, Daniel Persky, Ralph Martel, Constantine Sabalos, Bruce Seligmann, Rita Braziel, Elias Campo, Andreas Rosenwald, Joseph Connors, Laurie Sehn, Nathalie Johnson, and Randy Gascoyne (2008) "Gene expression predicts overall survival in paraffin embedded tissues of diffuse large B cell lymphoma treated with R-CHOP" Blood, 2008 Oct 15, 112 (8): 3425-33
5. Pechhold, S., Stouffer, M., Walker, G., Martel R., Seligmann, B, Hang, Y., Stein R., Harlan, DM., and Pechhold, K. (2009). mRNA analysis of intracytoplasmically-stained, FACS-purified pancreatic islet cell subsets using the quantitative nuclease protection assay. Nature Biotechnology, TR21220A.
6. Pino S, Ciciriello F, Costanzo G, and Di Mauro E (2008). Nonenzymatic RNA Litgation in Water. Journal of Biological Chemistry, Vol. 283: No.52: 26494-36503.
7. Lutay AV, Chernolovskaya EL, Zenkova MA, Vlassov VV (2006). The nonenzyatic template-directed ligation of oligonucleotides. Biosciences, 3, 243-249.
8. Shabarova ZA, Merenkova IN, Oretskaya TS, Sokolova NI, Skripkin EA, Alexeyeva EV, Balakin AG, Bogdanov (1991). Chemical ligation of DNA: the first non-enzymatic assembly of a biologically active gene. Nucleic Acids Research, Vol. 19: No. 15: 4247-4251.
9. United States Patent 7,033,753. Inventor: Kool, Eric T: Assignee: University of Rochester. Compositions and methods for nonenzymatic ligation of oligonucleotides and detection of genetic polymorphisms. April 25, 2006.
10. Banér J, Isaksson A, Waldenström E, Jarvius J, Landegren U, Nilsson M (2003). Parallel gene analysis with allele-specific padlock probes and tag microarrays. Nucleic Acids Research 31 (17):e103(1-7).
11. Prins TW, vanDijk JP, Beenen HG, Van Hoef AMA, Voorhuijzen MM, Schoen CD, Aarts HJM, Kok EJ (1008). Optimised padlock probe ligation and microarray detection of multiple (non-authorised) GMOs in single reaction. BMC Genomics 9:584(1-12).

## Claims

1. A method of targeted sequencing of at least one nucleic acid target in a biological sample comprising
(i) contacting said sample with at least one nuclease protection probe (NPP) comprising a nucleic acid molecule which specifically hybridizes to said target, under conditions that permit the hybridization of the NPP to the target nucleic acid,
(ii) exposing said sample to one or more nucleases or nuclease cocktails under conditions that are effective to eliminate any NPP that is not hybridized to the target molecule, leaving an amount of target/NPP duplex, and/or target /partial NPP duplex,
(iii) separating the hybridized NPP from the target,
(iV) contacting the NPP with at least one tag linker and a gene-specific or experiment tag in combination or separately, the tag linker comprising nucleic acid sequences complementary to a) the 3' end of the NPP and b) a portion of a sequence of the gene-specific or experiment tag, under conditions that permit the tag linker to hybridize to the NPP and the gene-specific or experiment tag to form a complete adduct,
V) ligating together the NPP and gene-specific or experiment tag sequences in the complete adduct to form a complete sequencible adduct, and
(vi) sequencing said sequencible adduct.
wherein the experiment tag is unique to the sample.

2. A method according to claim 1, wherein the gene-specific or experiment tag has been synthesized with a 3' adapter sequence.

3. A method according to claim 1, wherein the tag linker extends the full length of the NPP and further, and further comprises a nucleic acid sequence complementary to the 5' end of the NPP and an adapter sequence, and wherein step iv) of the method further comprises contacting the NPP with an adapter sequence under conditions that permit the tag linker to hybridize to the adapter sequence.

4. A method according to claim 1, further comprising contacting the NPP with at least one adapter linker which hybridizes to the 5' end of the NPP and comprises a nucleic sequence that is complementary to a 5' adapter sequence, and an adapter sequence under conditions that permit the adapter linker to hybridize to the NPP and the adapter sequence.

5. A method according to claim 1 wherein the target is fixed or cross-linked or insoluble, particularly wherein said insoluble nucleic acid is a cross-linked mRNA, miRNA, or vRNA.

6. A method according to claim 1 wherein said nucleic acid target molecule comprises a ribonucleic acid (RNA) molecule or a deoxyribonucleic (DNA) molecule, or an antisense nucleotide that optionally contains unnatural bases, particularly wherein said RNA is a messenger RNA (mRNA), a ribosomal RNA (rRNA), a transfer RNA (tRNA), micro RNA (miRNA), an siRNA, an anti-sense RNA, or a viral RNA (vRNA), particularly wherein said DNA is a genomic DNA (gDNA), mitochondrial DNA (mtDNA), chloroplast DNA (cpDNA), viral DNA (vDNA), a cDNA, or a transfected DNA.

7. A method according to claim 1 wherein said NPP comprises a DNA molecule, particularly wherein said NPP is a single stranded (ssDNA) or branched DNA (bDNA) molecule or an aptamer, or contains LNA or PNA or a polynucleotide which comprises unnatural bases.

8. A method according to claim 1 wherein said target is an RNA molecule, microRNA, siRNA or antisense RNA that optionally comprises unnatural bases, preferably wherein said target RNA molecule hybridizes to the complete NPP molecule or a portion thereof, or wherein said NPP is a single stranded (ssDNA) or a branched (bDNA) DNA, or wherein said one or more nucleases or nuclease cocktails is a DNAase, an RNAase, an endonuclease , an exonuclease, or a combination thereof, or wherein said NPP is a DNA molecule and said one or more nucleases or nuclease cocktails is a DNAase and an RNAase, or wherein said nuclease is an S1 nuclease, or wherein said one or more nucleases or nuclease cocktails is an exonuclease.

9. A method according to claim 1 wherein said biological sample is fixed, or wherein said biological sample comprises an agent that causes target molecule cross-linking, wherein said target is cross-linked.

10. A method according to claim 1, wherein sequencing comprises Solexa sequencing, 454 sequencing, chain termination sequencing, dye termination sequencing or pyrosequencing, or single molecule sequencing.

11. A method according to claim 1 wherein the target molecule is sequenced without extraction or solubilization, or further comprising biosynthetically producing an NPP using the target molecule as a template.

12. A method according to claim 1 further comprising amplifying said sequencibile adduct containing the NPP after step ii) but before step vi), preferably wherein the amplification processes comprises PCR amplification, optionally wherein said NPP comprises a nucleic acid which specifically binds to said target, or is comprised in part or entirely of peptide nucleic acids, or is comprised in part or entirely of LNAs, or unnatural bases, or modified bases.

13. The method of claim 1, wherein the method detects at least one target in a plurality of biological samples simultaneously, or wherein the method detects several targets in a biological sample, and wherein the sample is contacted with several NPPs, wherein each NPP specifically binds to a particular target.

14. The method of claim 1, further comprising:
comparing an obtained-sequence to a reference sequence database; and
counting each identified sequence.

15. A method of targeted sequencing of at least one target in a biological sample comprising
(i) contacting said sample with at least one linear nuclease protection probe (NPP), the ends of which specifically binds to said target such that the 5' and 3' end are hybridized to adjacent bases of the target, wherein the NPP further comprises a sequencing adapter and a gene-specific or experiment tag sequence,
(ii) ligating said NPP to form a circular oligonucleotide,
(iii) dissociating the circular NPP, hybridizing a second molecule of linear NPP to the target, and ligating,
(iv) optionally repeating (iii) in successive cycles,
(v) adding an exonuclease to destroy all linear single stranded oligonucleotide in the sample,
(vi) cleaving the circular NPP to linearize said NPP, and
(vii) sequencing the linear NPP,
wherein the experiment tag is unique to the sample.

## Patentansprüche

1. Verfahren zur targetierten Sequenzierung zumindest eines Nukleinsäure-Targets in einer biologischen Probe, umfassend
(i) Berühren besagter Probe mit zumindest einer Nukleaseschutz-Sonde (NPP) umfassend ein Nukleinsäuremolekül, das spezifisch an besagtes Target hybridisiert, unter Bedingungen, die das Hybridisieren der NPP an die Target-Nukleinsäure gestatten,
(ii) Aussetzen besagter Probe an eine oder mehrere Nukleasen oder Nuklease-Cocktails unter Bedingungen, die jede NPP, die nicht an das Targetmolekül hybridisiert ist, wirksam eliminieren, wobei eine Menge von Target/NPP-Duplex und/oder Target/partiellem NPP-Duplex hinterlassen wird,
(iii) Separieren der hybridisierten NPP vom Target,
(iv) Berühren der NPP mit zumindest einem Tag-Linker und einem genspezifischen oder Experiment-Tag zusammen oder separat, wobei der Tag-Linker Nukleinsäuresequenzen umfasst, die komplementär zu a) dem 3'-Ende der NPP und b) einem Teil einer Sequenz des genspezifischen oder Experiment-Tags sind, unter Bedingungen, die dem Tag-Linker das Hybridisieren an die NPP und das genspezifische oder Experiment-Tag, um ein vollständiges Addukt zu bilden, gestatten,
(v) Zusammenligieren der NPP und genspezifischen oder Experiment-Tag-Sequenzen im vollständigen Addukt, um ein vollständiges sequenzierbares Addukt zu bilden, und
(vi) Sequenzieren des besagten sequenzierbaren Addukts.
worin das Experiment-Tag einzigartig für die Probe ist.

2. Verfahren nach Anspruch 1, worin das genspezifische oder Experiment-Tag mit einer 3'-Adaptersequenz synthetisiert worden ist.

3. Verfahren nach Anspruch 1, worin der Tag-Linker sich über die volle Länge der NPP erstreckt und ferner eine Nukleinsäuresequenz umfasst, die komplementär zu dem 5'-Ende der NPP und einer Adaptersequenz ist, und worin Schritt (iv) des Verfahrens ferner das Berühren der NPP mit einer Adaptersequenz unter Bedingungen umfasst, die dem Tag-Linker das Hybridisieren an die Adaptersequenz gestatten.

4. Verfahren nach Anspruch 1, ferner umfassend das Berühren der NPP mit zumindest einem Adapterlinker, der an das 5'-Ende der NPP hybridisiert und eine Nukleinsäuresequenz umfasst, die komplementär zu einer 5'-Adaptersequenz und einer Adaptersequenz unter Bedingungen ist, die dem Adapterlinker das Hybridisieren an die NPP und die Adaptersequenz gestatten.

5. Verfahren nach Anspruch 1, worin das Target fixiert oder vernetzt oder unlöslich ist, insbesondere, worin besagte unlösliche Nukleinsäure eine vernetzte mRNA, miRNA oder vRNA ist.

6. Verfahren nach Anspruch 1, worin besagtes Nukleinsäure-Targetmolekül ein Ribonukleinsäure- (RNA-) Molekül oder ein Desoxyribonukleinsäure- (DNA-) Molekül oder ein Antisense-Nukleotid umfasst, das wahlweise unnatürliche Basen enthält, insbesondere, worin besagte RNA eine Messenger-RNA (mRNA), eine ribosomale RNA (rRNA), eine Transfer-RNA (tRNA), Mikro-RNA (miRNA), eine siRNA, eine Antisense-RNA oder eine virale RNA (vRNA) ist, insbesondere, worin besagte DNA eine genomische DNA (gDNA), mitochondriale DNA (mtDNA), Chloroplasten-DNA (cpDNA), virale DNA (vDNA), eine cDNA oder eine transfizierte DNA ist.

7. Verfahren nach Anspruch 1, worin besagte NPP ein DNA-Molekül umfasst, insbesondere, worin besagte NPP ein einzelsträngiges (ssDNA) oder verzweigtes DNA- (bDNA) Molekül oder ein Aptamer ist oder LNA oder PNA oder ein Polynukleotid, die oder das unnatürliche Basen umfasst, enthält.

8. Verfahren nach Anspruch 1, worin besagtes Target ein RNA-Molekül, microRNA, siRNA oder Antisense-RNA ist, das oder die wahlweise unnatürliche Basen enthält sind, vorzugsweise, worin besagtes Target-RNA-Molekül an das vollständige NPP-Molekül oder einen Teil davon hybridisiert, oder worin besagte NPP eine einzelsträngige (ssDNA) oder eine verzweigte (bDNA) DNA ist, oder worin es sich bei besagter einer oder mehreren Nukleasen oder Nuklease-Cocktails um eine DNAase, eine RNAase, eine Endokuklease, eine Exonuklease oder eine Kombination davon handelt, oder worin besagte NPP ein DNA-Molekül ist und es sich bei besagter einer oder mehreren Nukleasen oder Nuklease-Cocktails um eine DNAase und eine RNAase handelt, oder worin besagte Nuklease eine S1-Nuklease ist, oder worin es sich bei besagter einer oder mehreren Nukleasen oder Nuklease-Cocktails um eine Exonuklease handelt.

9. Verfahren nach Anspruch 1, worin besagte biologische Probe fixiert ist oder worin besagte biologische Probe ein Mittel umfasst, das Targetmolekül-Vernetzung bewirkt, worin besagtes Target vernetzt ist.

10. Verfahren nach Anspruch 1, worin die Sequenzierung eine Solexa-Sequenzierung, 454-Sequenzierung, Kettenabbruch-Sequenzierung, Dye-Termination-Sequenzierung oder Pyrosequenzierung oder Einzelmolekül-Sequenzierung umfasst.

11. Verfahren nach Anspruch 1, worin das Targetmolekül ohne Extraktion oder Solubilisierung sequenziert wird, oder ferner umfassend die biosynthetische Erzeugung einer NPP mithilfe des Targetmoleküls als Schablone.

12. Verfahren nach Anspruch 1, ferner umfassend Amplifikation des besagten sequenzierbaren Addukts enthaltend die NPP nach Schritt (ii), aber vor Schritt (vi), vorzugsweise, worin die Amplifikationsprozesse eine PCR-Amplifikation umfassen, wahlweise, worin besagte NPP eine Nukleinsäure umfasst, die spezifisch an besagtes Target bindet, oder teilweise oder gänzlich aus Peptid-Nukleinsäuren besteht, oder teilweise oder gänzlich aus LNAs oder unnatürlichen Basen oder modifizierten Basen besteht.

13. Verfahren nach Anspruch 1, worin das Verfahren zumindest ein Target in einer Mehrzahl von biologischen Probe gleichzeitig nachweist, oder worin das Verfahren mehrere Targets in einer biologischen Probe nachweist, und worin die Probe mit mehreren NPPs berührt wird, worin jede NPP spezifisch an ein bestimmtes Target bindet.

14. Verfahren nach Anspruch 1, ferner umfassend:
Vergleich einer erhaltenen Sequenz mit einer Referenzsequenzdatenbank; und
Zählen jeder identifizierten Sequenz.

15. Verfahren zur targetierten Sequenzierung zumindest eines Targets in einer biologischen Probe, umfassend
(i) Berühren besagter Probe mit zumindest einer linearen Nukleaseschutz-Sonde (NPP), deren Enden spezifisch an besagtes Target binden, sodass das 5'- und das 3'-Ende an angrenzende Basen des Targets hybridisiert werden, worin die NPP ferner einen Sequenzierungsadapter und eine genspezifische oder Experiment-Tag-Sequenz umfasst,
(ii) Ligieren besagter NPP, um ein kreisförmiges Oligonukleotid zu bilden,
(iii) Dissoziieren der kreisförmigen NPP, Hybridisieren eines zweiten Moleküls von linearer NPP an das Target, und Ligieren,
(iv)wahlweises Wiederholen von (iii) in sukzessiven Zyklen,
(v) Hinzufügen einer Exonuklease, um alle linearen einzelsträngigen Oligonukleotide in
der Probe zu zerstören,
(vi) Spalten der kreisförmigen NPP, um besagte NPP zu linearisieren, und
(vii) Sequenzieren der linearen NPP,
worin das Experiment-Tag einzigartig für die Probe ist.

## Revendications

1. Procédé de séquençage ciblé d'au moins une cible d'acide nucléique dans un échantillon biologique, consistant à :
(i) mettre en contact ledit échantillon avec au moins une sonde de protection contre la nucléase (NPP) comprenant une molécule d'acide nucléique qui s'hybride de manière spécifique avec ladite cible, dans des conditions qui permettent l'hybridation de la NPP avec l'acide nucléique cible ;
(ii) exposer ledit échantillon à une ou plusieurs nucléases ou à un ou plusieurs cocktails de nucléases dans des conditions qui sont efficaces pour éliminer toute NPP qui n'est pas hybridée avec la molécule cible, de manière à laisser une quantité d'un duplex cible/NPP, et/ou d'un duplex cible/NPP partielle ;
(iii) séparer la NPP hybrider de la cible ;
(iv) mettre en contact la NPP avec au moins un marqueur de liaison et un marqueur spécifique d'un gène ou d'expérimentation en combinaison ou indépendamment, le marqueur de liaison comprenant des séquences d'acides nucléiques complémentaires à a) la terminaison 3' de la NPP et b) une partie d'une séquence du marqueur spécifique d'un gène ou d'expérimentation, dans des conditions qui permettent au marqueur de liaison de s'hybrider avec la NPP et le marqueur spécifique d'un gène ou d'expérimentation afin de former un adduit complet ;
(v) ligaturer ensemble les séquences de NPP et de marqueur spécifique d'un gène ou d'expérimentation dans l'adduit complet afin de former un adduit séquençable complet ; et
(vi) séquencer ledit conduit séquençable ;
dans lequel le marqueur d'expérimentation est propre à l'échantillon.

2. Procédé selon la revendication 1, dans lequel le marqueur spécifique d'un gène ou d'expérimentation a été synthétisé avec une séquence adaptatrice 3'.

3. Procédé selon la revendication 1, dans lequel le marqueur de liaison prolonge la longueur totale de la NPP, et comprend en outre une séquence d'acides nucléiques complémentaire de la terminaison 5' de la NPP et une séquence adaptatrice, et l'étape iv) du procédé consistant en outre à mettre en contact la NPP avec une séquence adaptatrice dans des conditions qui permettent au marqueur de liaison de s'hybrider avec la séquence adaptatrice.

4. Procédé selon la revendication 1, consistant en outre à mettre en contact la NPP avec au moins un adaptateur de liaison qui s'hybride avec la terminaison 5' de la NPP et comprend une séquence d'acides nucléiques complémentaire d'une séquence adaptatrice 5', et une séquence adaptatrice dans des conditions qui permettent à l'adaptateur de liaison de s'hybrider avec la NPP et la séquence adaptatrice.

5. Procédé selon la revendication 1, dans lequel la cible est fixe, réticulée ou insoluble, en particulier dans lequel ledit acide nucléique insoluble est un ARNm, un ARNmi ou un ARNv.

6. Procédé selon la revendication 1, dans lequel la molécule cible d'acide nucléique comprend une molécule d'acide ribonucléique (ARN) ou une molécule d'acide désoxyribonucléique (ADN), ou un nucléotide antisens qui contient éventuellement des bases non naturelles, en particulier dans lequel l'ARN est un ARN messager (ARNm), un ARN ribosomique (ARNr), un ARN de transfert (ARNt), un ARN micro (ARNmi), un ARNsi, un ARN antisens ou un ARN viral (ARNv), en particulier dans lequel ledit ADN est un ADN génomique (ADNg), un ADN mitochondrial (ADNmt), un ADN du chloroplaste (ADNcp), un ADN viral (ADNv), un ADNc ou un ADN transfecté.

7. Procédé selon la revendication 1, dans lequel la NPP comprend une molécule d'ADN, en particulier dans lequel ladite NPP est une molécule d'ADN à simple brin (ADNss) ou d'ADN ramifié (ADNb) ou un aptamère, ou contient un acide LNA ou PNA ou un polynucléotide qui comprend des bases non naturelles.

8. Procédé selon la revendication 1, dans lequel ladite cible est une molécule d'ARN, un ARN micro, un ARNsi ou un ARN antisens qui comprend éventuellement des bases non naturelles, de préférence dans lequel ladite molécule d'ARN cible s'hybride avec la molécule NPP complète ou une partie correspondante, ou dans lequel ladite NPP est un ADN à simple brin (ADNss) ou ramifié (ADNb), ou dans lequel ladite ou lesdites nucléases ou ledit ou lesdits cocktails de nucléases sont une ADNase, une ARNase, une endonucléase, une exonucléase ou une combinaison correspondante, ou dans lequel ladite NPP est une molécule d'ADN et ladite ou lesdites nucléases ou ledit ou lesdits cocktails de nucléases sont une ADNase et une ARNase, ou dans lequel ladite nucléase est une nucléase S1, ou dans lequel ladite ou lesdites nucléases ou ledit ou lesdits cocktails de nucléases sont une exonucléase.

9. Procédé selon la revendication 1, dans lequel ledit échantillon biologique est fixé, ou dans lequel l'échantillon biologique comprend un agent qui provoque la réticulation des molécules cibles, ladite cible étant réticulée.

10. Procédé selon la revendication 1, dans lequel le séquençage un séquençage Solexa, un séquençage 454, un séquençage à terminaison de chaîne, un séquençage à terminaison-colorant, un pyroséquençage ou un séquençage à partir de molécules uniques.

11. Procédé selon la revendication 1, dans lequel la molécule cible est séquencée sans extraction ou solubilisation, ou consistant en outre à produire de manière biosynthétique une NPP à l'aide de la molécule cible en guise de matrice.

12. Procédé selon la revendication 1, consistant en outre à amplifier ledit adduit séquençable contenant la NPP après l'étape ii) mais avant l'étape vi), de préférence dans lequel les procédés d'amplification comprennent une amplification par PCR, éventuellement dans lequel ladite NPP comprend un acide nucléique qui se lie de manière spécifique à ladite cible, ou est constituée en partie ou totalement d'acides nucléiques peptidiques, ou est constituée en partie ou totalement d'acides LNA ou de bases non naturelles ou de bases modifiées.

13. Procédé selon la revendication 1, dans lequel le procédé détecte simultanément au moins une cible dans une pluralité d'échantillons biologiques, ou le procédé détectant plusieurs cibles dans un échantillon biologique, et dans lequel l'échantillon est mis en contact avec plusieurs NPP, chaque NPP se liant de manière spécifique à une cible particulière.

14. Procédé selon la revendication 1, consistant en outre à :
comparer une séquence obtenue à une base de données de séquences de référence ; et
compter chaque séquence identifiée.

15. Procédé de séquençage ciblé d'au moins une cible dans un échantillon biologique, consistant à :
(i) mettre en contact ledit échantillon avec au moins une sonde linéaire de protection contre la nucléase (NPP), dont les terminaisons se lient de manière spécifique à ladite cible, de telle sorte que les terminaisons 5' et 3' s'hybrident avec des bases adjacentes de la cible, la NPP comprenant en outre un adaptateur de séquençage et une séquence de marqueurs spécifiques d'un gène ou d'expérimentation ;
(ii) ligaturer ladite NPP pour former un oligonucléotide circulaire ;
(iii) dissocier la NPP circulaire, hybrider une seconde molécule de NPP linéaire à la cible, et ligaturer ;
(iv) éventuellement répéter l'étape (iii) en cycles successifs ;
(v) ajouter une exonucléase pour détruire tous les oligonucléotides à simple brin linéaires de l'échantillon ;
(vi) cliver la NPP circulaire pour linéariser ladite NPP ; et
(vii) séquencer la NPP linéaire ;
dans lequel le marqueur d'expérimentation est propre à l'échantillon.
